(19) Europäisches Patentamt
European Patent Office
Office européen des brevets

(11) EP 3 546 488 A1

(12) **EUROPEAN PATENT APPLICATION**
published in accordance with Art. 153(4) EPC

(43) Date of publication:
**02.10.2019 Bulletin 2019/40**

(21) Application number: **17874134.4**

(22) Date of filing: **10.11.2017**

(51) Int Cl.:
***C08F 20/34*** *(2006.01)*
***C08F 2/48*** *(2006.01)*
***C08F 20/38*** *(2006.01)*
***C08F 220/34*** *(2006.01)*
***C08F 220/38*** *(2006.01)*
***G02B 1/11*** *(2015.01)*
***G02B 5/30*** *(2006.01)*

(86) International application number:
**PCT/JP2017/040656**

(87) International publication number:
**WO 2018/096958 (31.05.2018 Gazette 2018/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.11.2016 JP 2016227306**

(71) Applicant: **Zeon Corporation**
**Tokyo 100-8246 (JP)**

(72) Inventors:
- **SAKAMOTO, Kei**
  **Tokyo 100-8246 (JP)**
- **OKUYAMA, Kumi**
  **Tokyo 100-8246 (JP)**

(74) Representative: **Adam, Holger et al**
**Kraus & Weisert**
**Patentanwälte PartGmbB**
**Thomas-Wimmer-Ring 15**
**80539 München (DE)**

(54) **POLYMERIZABLE COMPOUND, MIXTURE, POLYMERIZABLE LIQUID CRYSTAL COMPOSITION, POLYMER, OPTICAL FILM, OPTICALLY ANISOTROPIC BODY, POLARIZATION PLATE, DISPLAY DEVICE AND ANTIREFLECTION FILM**

(57) The objective is to enable production of an optical film having good reverse wavelength dispersion. A mixture contains polymerizable compounds indicated by formulae (I) and (V), shown below. In the formulae: $Ar^1$ and $Ar^2$ are each a prescribed heterocyclic group; $A^1$ to $A^4$ and $B^1$ to $B^4$ are each an optionally substituted alicyclic group or aromatic group; $Y^1$ to $Y^4$, $L^1$ to $L^4$, and $Z^1$ to $Z^4$ are each a prescribed group such as -O-, -CO-O-, or -O-CO-; $R^1$ to $R^6$ are each a hydrogen atom, a methyl group, or a chlorine atom; one of e and f is an integer of 1 to 3 and the other of e and f is an integer of 0 to 3; c, d, i, and j are each an integer of 1 to 20; and a, b, g, and h are each 0 or 1.

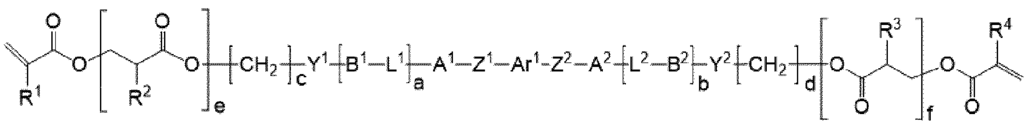

( I )

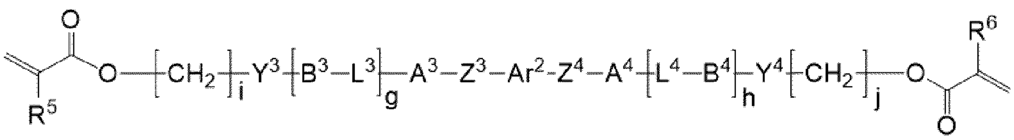

( V )

EP 3 546 488 A1

FIG. 2A

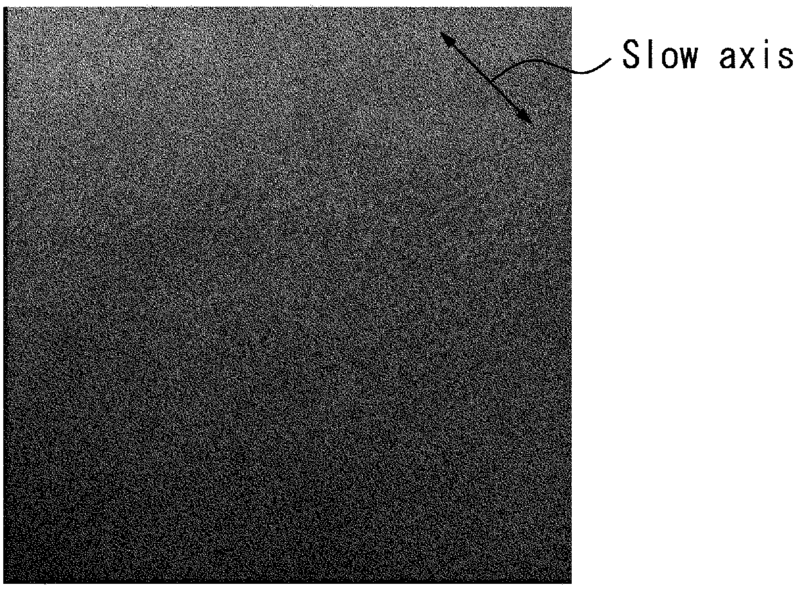


FIG. 2B

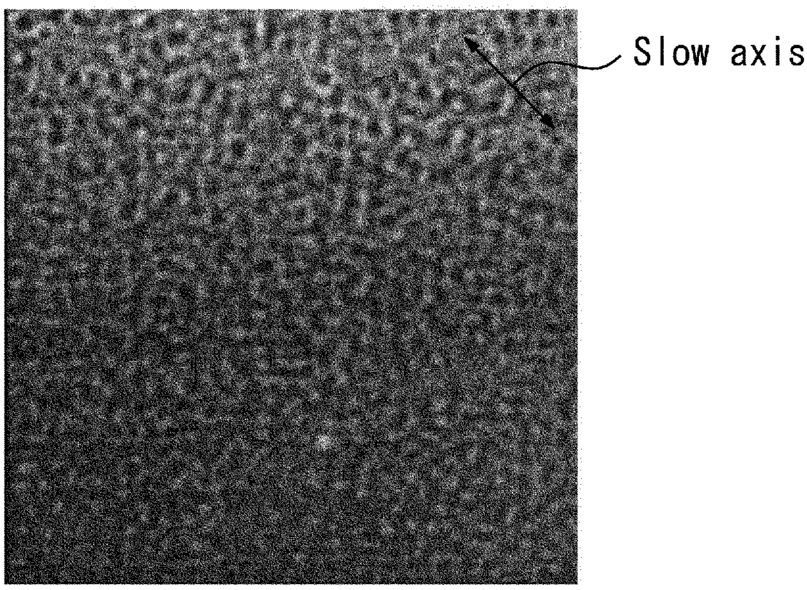

**Description**

TECHNICAL FIELD

The present disclosure relates to an optical film and an optically anisotropic body that can perform uniform polarized light conversion over a wide wavelength region, and to a polarizer, a display, and an antireflection film in which the optically anisotropic body is used.

Moreover, the present disclosure relates to a polymer that can be used in production of the optical film and the optically anisotropic body, and to a polymerizable compound and a mixture and polymerizable liquid crystal composition containing the polymerizable compound that can be used in production of the polymer.

BACKGROUND

Examples of retardation plates used in various devices such as flat panel displays include quarter-wave plates that convert linearly polarized light to circularly polarized light and half-wave plates that perform 90° conversion of the plane of vibration of linearly polarized light. Such retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. Specifically, since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment to polarized light with a retardation of 1/4λ or 1/2λ over the entire wavelength region of input light.

In order to solve this problem, various retardation plates having a property referred to as "reverse wavelength dispersion" have been studied. These retardation plates are wideband retardation plates that can achieve uniform retardation with respect to light over a wide wavelength region.

On the other hand, enhanced functionality and widespread use of mobile information terminals such as mobile personal computers and mobile phones has been accompanied by demand for thickness-reduction of flat panel displays to as great an extent as possible. Consequently, there has also been demand for thickness-reduction of retardation plates used as components thereof.

In terms of methods of achieving thickness-reduction, a method in which a retardation plate is produced by applying a polymerizable composition containing a low-molecular weight polymerizable compound onto a film substrate to form an optical film has been regarded as the most effective method in recent years. For this reason, there has been much development of polymerizable compounds that are capable of forming optical films that excel in terms of reverse wavelength dispersion, and also polymerizable compositions in which these compounds are used.

For example, PTL 1 and 2 propose polymerizable compounds and polymerizable compositions that are capable of forming optical films having excellent reverse wavelength dispersion, have low melting points suitable for processing, can easily be applied onto substrates, display liquid crystallinity over wide temperature ranges, and can be cheaply synthesized.

CITATION LIST

Patent Literature

PTL 1: WO 2014/010325 A1
PTL 2: JP 2015-200877 A

SUMMARY

(Technical Problem)

In industrial-scale production of an optical film or optically anisotropic body (hereinafter, also referred to collectively as an "optical film or the like") using a polymerizable composition containing a polymerizable compound, wide production condition tolerance (process margin) is required.

Particularly in a case in which the polymerizable composition is to be applied over a wide area to produce an optical film or the like, it is difficult to achieve complete uniformity in terms of temperature in a drying furnace and time

conditions. Consequently, the yield of the optical film or the like is significantly influenced by tolerance with respect to production conditions such as temperature and time.

However, when conventional polymerizable compounds and polymerizable compositions have been used, an optical film or the like that can maintain a liquid crystal phase over a long period with higher stability has not been obtained, and the process margin has been inadequate. Consequently, there is demand for a polymerizable liquid crystal composition containing a polymerizable compound with which an optical film or the like that can maintain a liquid crystal phase over a long period with higher stability can be formed.

The present disclosure was completed in view of the circumstances set forth above and has an objective of providing a polymerizable liquid crystal composition with which it is possible to form an optical film or the like that has a practical low melting point, can be produced at low cost, can perform uniform polarized light conversion over a wide wavelength region, and can stably maintain a liquid crystal phase over a long period.

Another objective of the present disclosure is to provide a polymerizable compound and a mixture containing the polymerizable compound that are useful in production of the polymerizable liquid crystal composition.

(Solution to Problem)

As a result of diligent research conducted in order to solve the problem set forth above, the inventors discovered that by using a mixture of a specific polymerizable compound indicated by formula (I), shown below, and a specific polymerizable compound indicated by formula (V), shown below, it is possible to obtain, at low cost, a polymerizable liquid crystal composition with which an optical film or the like that can maintain a liquid crystal phase over a long period with higher stability, has little coating unevenness, and has excellent reverse wavelength dispersion can be formed, and in this manner completed the present disclosure.

Accordingly, the present disclosure provides the following polymerizable compound, mixture, polymerizable liquid crystal composition, polymer, optical film, optically anisotropic body, polarizer, display, and antireflection film.

[1] A polymerizable compound indicated by formula (I), shown below,

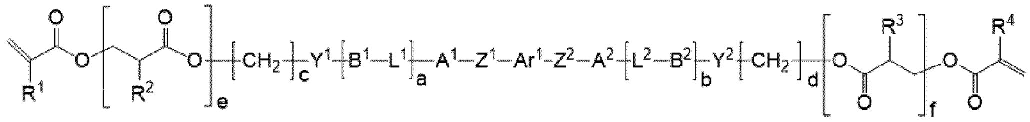

( I )

where, in formula (I), $Ar^1$ is any one of groups represented by formulae (II-1) to (II-4), shown below,

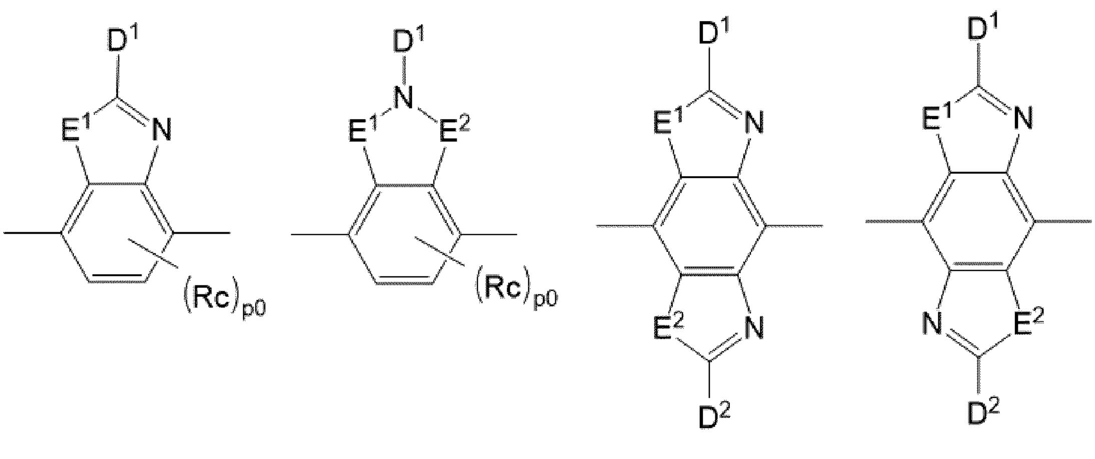

(II-1) (II-2) (II-3) (II-4)

$E^1$ and $E^2$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number of 1 to 4,

Rc represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon

number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,

p0 is an integer of 0 to 2,

$D^1$ and $D^2$ each represent, independently of one another, an optionally substituted aromatic hydrocarbon cyclic group or an optionally substituted aromatic heterocyclic group,

$Z^1$ and $Z^2$ each represent, independently of one another, a single bond, $-O-CH_2-$, $-CH_2-O-$, -C(=O)-O-, -O-C(=O)-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, where $R^{13}$ represents a hydrogen atom or an alkyl group having a carbon number of 1 to 6,

$A^1$, $A^2$, $B^1$, and $B^2$ each represent, independently of one another, an optionally substituted alicyclic group or an optionally substituted aromatic group,

$Y^1$, $Y^2$, $L^1$, and $L^2$ each represent, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$, where $R^{14}$ and $R^{15}$ each represent, independently of one another, a hydrogen atom or an alkyl group having a carbon number of 1 to 6,

$R^1$ to $R^4$ each represent, independently of one another, a hydrogen atom, a methyl group, or a chlorine atom,

a and b are each, independently of one another, 0 or 1,

one of e and f is an integer of 1 to 3 and the other of e and f is an integer of 0 to 3,

c and d are each, independently of one another, an integer of 1 to 20, and

in a case in which more than one $R^2$, $R^3$, or Rc is present, each $R^2$, $R^3$, or Rc may be the same or different.

[2] The polymerizable compound according to the foregoing [1], wherein $D^1$ and $D^2$ are each, independently of one another, any one of groups represented by formulae (III-1) to (III-8), shown below,

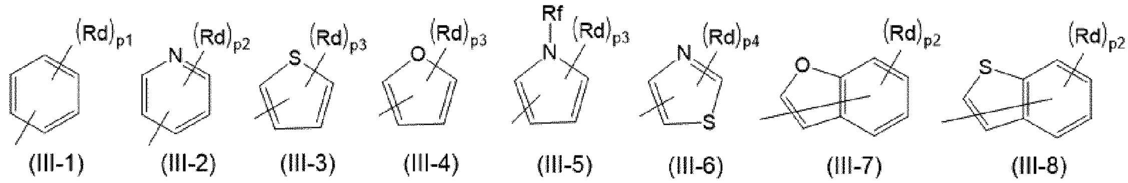

(III-1) (III-2) (III-3) (III-4) (III-5) (III-6) (III-7) (III-8)

where, in formulae (III-1) to (III-8), Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,

p1 represents an integer of 0 to 5, p2 represents an integer of 0 to 4, p3 represents an integer of 0 to 3, and p4 represents an integer of 0 to 2,

Rf represents a hydrogen atom or a methyl group, and

in a case in which more than one Rd is present, each Rd may be the same or different.

[3] The polymerizable compound according to the foregoing [1] or [2], wherein $Ar^1$ is any one of groups represented by formulae (IV-1) to (IV-5), shown below,

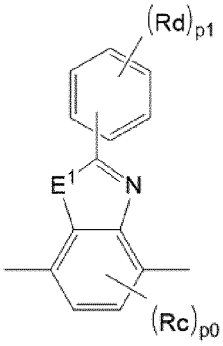

(IV-1)

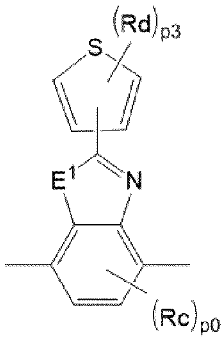

(IV-2)

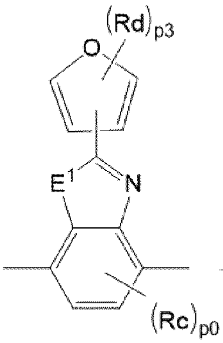

(IV-3)

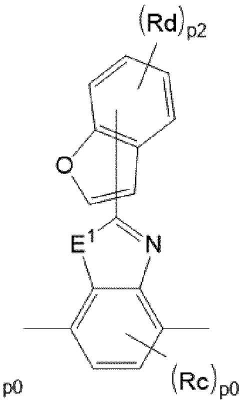

(IV-4)

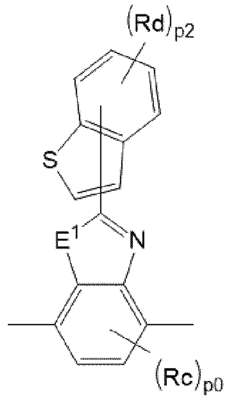

(IV-5)

where, in formulae (IV-1) to (IV-5), $E^1$, Rc, and p0 have the same meaning as previously described,
Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, and p3 represents an integer of 0 to 3, and in a case in which more than one Rc or Rd is present, each Rc or Rd may be the same or different.

[4] A mixture comprising:

the polymerizable compound according to any one of the foregoing [1] to [3]; and
a polymerizable compound indicated by formula (V), shown below,

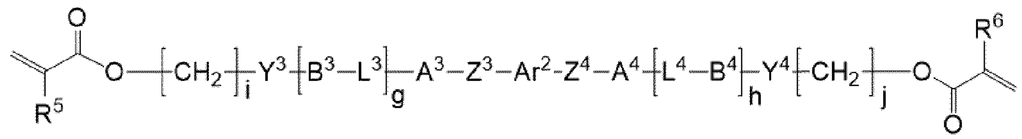

(V)

where, in formula (V), $Ar^2$ is any one of groups represented by formulae (VI-1) to (VI-4), shown below,

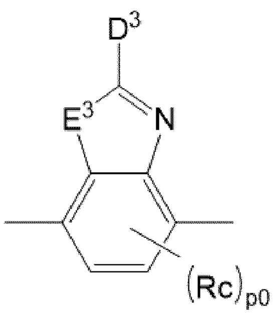

(VI-1)

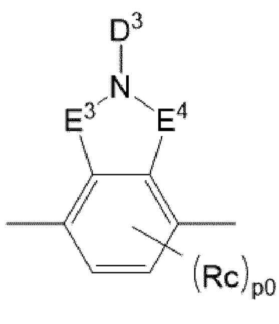

(VI-2)

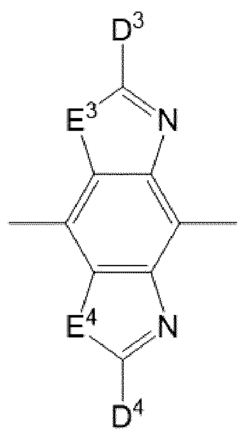

(VI-3)

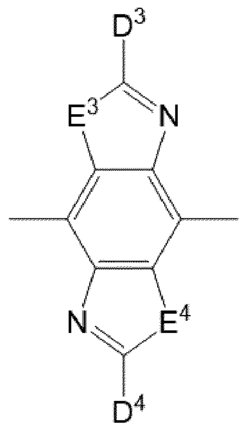

(VI-4)

$E^3$ and $E^4$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number

of 1 to 4,

Rc represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,

p0 is an integer of 0 to 2,

$D^3$ and $D^4$ each represent, independently of one another, an optionally substituted aromatic hydrocarbon cyclic group or an optionally substituted aromatic heterocyclic group,

$Z^3$ and $Z^4$ each represent, independently of one another, a single bond, $-O-CH_2-$, $-CH_2-O-$, -C(=O)-O-, -O-C(=O)-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, where $R^{13}$ represents a hydrogen atom or an alkyl group having a carbon number of 1 to 6,

$A^3$, $A^4$, $B^3$, and $B^4$ each represent, independently of one another, an optionally substituted alicyclic group or an optionally substituted aromatic group,

$Y^3$, $Y^4$, $L^3$, and $L^4$ each represent, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$, where $R^{14}$ and $R^{15}$ each represent, independently of one another, a hydrogen atom or an alkyl group having a carbon number of 1 to 6,

$R^5$ and $R^6$ each represent, independently of one another, a hydrogen atom, a methyl group, or a chlorine atom,

g and h are each, independently of one another, 0 or 1,

i and j are each, independently of one another, an integer of 1 to 20, and

in a case in which more than one Rc is present, each Rc may be the same or different.

[5] The mixture according to the foregoing [4], wherein $D^3$ and $D^4$ are each, independently of one another, any one of groups represented by formulae (III-1) to (III-8), shown below,

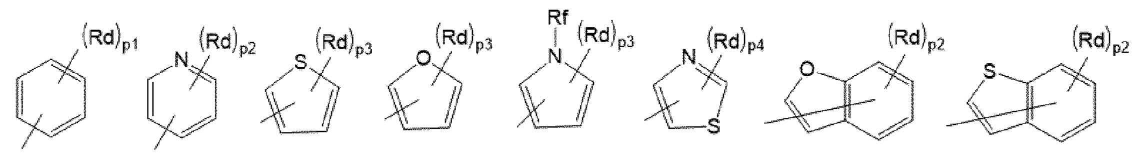

(III-1) (III-2) (III-3) (III-4) (III-5) (III-6) (III-7) (III-8)

where, in formulae (III-1) to (III-8), Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,

p1 represents an integer of 0 to 5, p2 represents an integer of 0 to 4, p3 represents an integer of 0 to 3, and p4 represents an integer of 0 to 2,

Rf represents a hydrogen atom or a methyl group, and

in a case in which more than one Rd is present, each Rd may be the same or different.

[6] The mixture according to the foregoing [4] or [5], wherein $Ar^2$ is any one of groups represented by formulae (VII-1) to (VII-5), shown below,

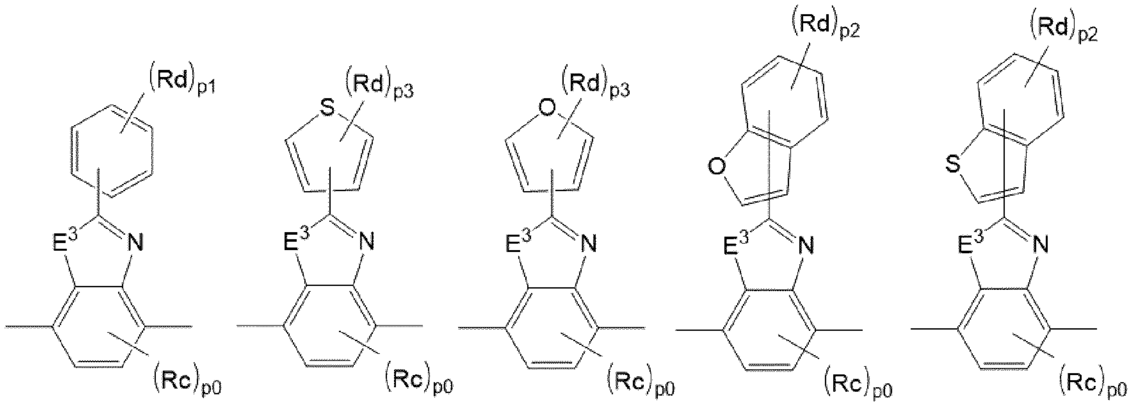

(VII-1) (VII-2) (VII-3) (VII-4) (VII-5)

where, in formulae (VII-1) to (VII-5), $E^3$, Rc, and p0 have the same meaning as previously described,
Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, and p3 represents an integer of 0 to 3, and in a case in which more than one Rc or Rd is present, each Rc or Rd may be the same or different.

[7] The mixture according to any one of the foregoing [4] to [6], wherein a mass ratio of the polymerizable compound indicated by formula (I) relative to the polymerizable compound indicated by formula (V) (polymerizable compound indicated by formula (I):polymerizable compound indicated by formula (V)) is 1:1,000 to 20:100.
[8] A polymerizable liquid crystal composition comprising:

the mixture according to any one of the foregoing [4] to [7]; and
a polymerization initiator.

[9] A polymer obtained by polymerizing the mixture according to any one of the foregoing [4] to [7] or the polymerizable liquid crystal composition according to the foregoing [8].
[10] An optical film comprising the polymer according to the foregoing [9] as a constituent material.
[11] An optically anisotropic body comprising a layer having the polymer according to the foregoing [9] as a constituent material.
[12] A polarizer comprising:

the optically anisotropic body according to the foregoing [11]; and
a polarizing film.

[13] A display comprising the polarizer according to the foregoing [12].
[14] An antireflection film comprising the polarizer according to the foregoing [12].

(Advantageous Effect)

The present disclosure provides a polymerizable liquid crystal composition with which an optical film or the like that can maintain a liquid crystal phase over a long period with higher stability, has a practical low melting point, and can perform uniform polarized light conversion over a wide wavelength region can be produced at low cost and with a wide process margin.
Moreover, the present disclosure provides a polymerizable compound and a mixture containing the polymerizable compound that are useful in production of the polymerizable liquid crystal composition.
Furthermore, the present disclosure provides an optical film and an optically anisotropic body that can perform uniform polarized light conversion over a wide wavelength region, and also provides a polarizer, a display, and an antireflection film in which the optical film and the optically anisotropic body are used.

BRIEF DESCRIPTION OF THE DRAWINGS

In the accompanying drawings:

FIG. 1A is a cross-sectional view illustrating configuration of a laminate used in a liquid crystal phase stability evaluation test;
FIG. 1B illustrates a relationship between an absorption axis and a slow axis for the laminate used in the liquid crystal phase stability evaluation test;
FIG. 2A is a photograph of a laminate used in a liquid crystal phase stability evaluation test that was taken from an opposite side to a light box side and that illustrates a case in which there was not non-uniformity in an optically anisotropic body of the laminate (evaluation index: 5); and
FIG. 2B is a photograph of a laminate used in a liquid crystal phase stability evaluation test that was taken from an opposite side to a light box side and that illustrates a case in which non-uniformity arose in an optically anisotropic body of the laminate (evaluation index: 1).

DETAILED DESCRIPTION

The following provides a detailed description of the present disclosure. Note that the phrase "optionally substituted" as used in the present disclosure means "unsubstituted or having one or more substituents". Also note that in a case in which an organic group (for example, an alkyl group or an aromatic hydrocarbon cyclic group) included in a general formula has a substituent, the carbon number of the organic group having the substituent is taken to be exclusive of the carbon number of the substituent. For example, in a case in which an aromatic hydrocarbon cyclic group having a carbon number of 6 to 20 has a substituent, the carbon number of the aromatic hydrocarbon cyclic group having a carbon number of 6 to 20 is taken to be exclusive of the carbon number of the substituent.

A presently disclosed polymerizable compound and a presently disclosed mixture containing the polymerizable compound can be used in production of a presently disclosed polymerizable liquid crystal composition, for example, but are not specifically limited to being used in this manner.

Moreover, the presently disclosed mixture and the presently disclosed polymerizable liquid crystal composition can be used in production of a presently disclosed polymer, for example, but are not specifically limited to being using in this manner.

The presently disclosed polymer can be used as a constituent material of a presently disclosed optical film or as a constituent material of a layer included in a presently disclosed optically anisotropic body, for example, but is not specifically limited to being used in this manner. Moreover, the presently disclosed optically anisotropic body can be used in a presently disclosed polarizer, for example, but is not specifically limited to being used in this manner. Furthermore, the presently disclosed polarizer can be used in a display (for example, a flat panel display or an organic electroluminescence display) or an antireflection film, for example, but is not specifically limited to being used in this manner.

(1) Polymerizable compound

The presently disclosed polymerizable compound is a compound indicated by the following formula (I) (hereinafter, also referred to as polymerizable compound (I)) and can advantageously be used in production of a polymer, an optical film, and an optically anisotropic body described further below.

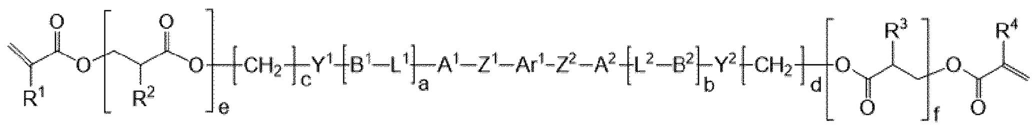

( I )

Note that when a mixture containing the polymerizable compound (I) and a polymerizable compound (V) (compound indicated by formula (V)), which is described further below in detail, is used as set forth further below, it is possible to obtain a polymerizable liquid crystal composition with which an optical film or the like that can maintain a liquid crystal phase over a long period with higher stability, has a wide process margin, has a practical low melting point, has excellent solubility in general purpose solvents, and can perform uniform polarized light conversion over a wide wavelength region can be produced at low cost.

Although the reason for this is not clear, it is presumed that as a result of the polymerizable compound (I) having a structure indicated by $-(CH_2CHR^2COO)_e-$ and/or $-(OCOCHR^3CH_2)_f-$, it is possible to ensure optical properties (particularly reverse wavelength dispersion) while forming a liquid crystal layer that readily adopts a liquid crystal phase at

lower temperature (i.e., readily adopts a supercooled state at room temperature) and obtaining an optical film or the like having a polymer as a constituent material using a mixture of the polymerizable compound (I) and the polymerizable compound (V), as compared to a case in which only the polymerizable compound (V) is used.

It should be noted that the polymerizable compound (I) may be used individually in production of a polymerizable liquid crystal composition, a polymer, and an optical film or the like having a polymer as a constituent material, without mixing the polymerizable compound (I) with the polymerizable compound (V).

In formula (I), a and b are each, independently of one another, 0 or 1, and preferably 1. Moreover, c and d are each, independently of one another, an integer of 1 to 20, preferably an integer of 2 to 12, and more preferably an integer of 4 to 8. Furthermore, one of e and f is an integer of 1 to 3 and the other of e and f is an integer of 0 to 3.

$Ar^1$ is any one of groups represented by the following formulae (II-1) to (II-4).

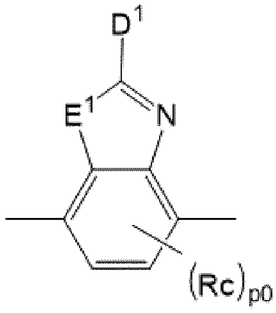

(II-1)

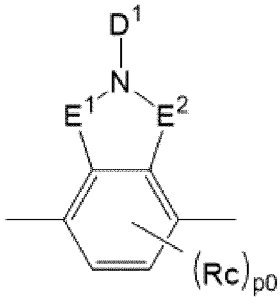

(II-2)

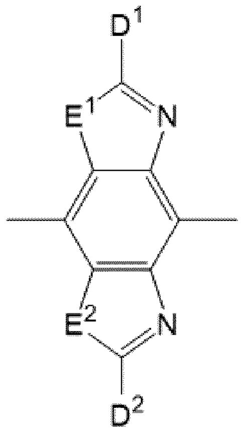

(II-3)

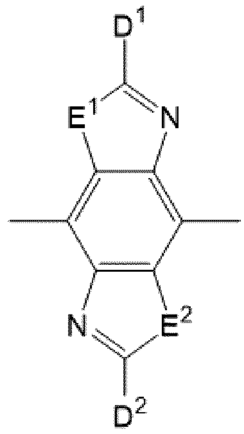

(II-4)

In formulae (II-1) to (II-4), p0 is an integer of 0 to 2, and preferably 0 or 1.

Rc represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12.

In a case in which more than one Rc is present in any of formulae (II-1) to (II-4) (i.e., in a case in which p0 is 2), each Rc may be the same or different.

The halogen atom of Rc may be a fluorine atom, a chlorine atom, a bromine atom, an iodine atom, or the like. Of these examples, a fluorine atom, a chlorine atom, and a bromine atom are preferable.

The alkyl group having a carbon number of 1 to 6 of Rc may be a methyl group, an ethyl group, an n-propyl group, an isopropyl group, an n-butyl group, an isobutyl group, a sec-butyl group, a tert-butyl group, a pentyl group, a hexyl group, or the like, is preferably an alkyl group having a carbon number of 1 to 4, and is particularly preferably a tert-butyl group or a methyl group. Moreover, the alkyl group of Rc mentioned above is preferably a chain alkyl group.

The alkylsulfinyl group having a carbon number of 1 to 6 of Rc may be a methylsulfinyl group, an ethylsulfinyl group, a propylsulfinyl group, an isopropylsulfinyl group, a butylsulfinyl group, an isobutylsulfinyl group, a sec-butylsulfinyl group, a tert-butylsulfinyl group, a pentylsulfinyl group, a hexylsulfinyl group, or the like, is preferably an alkylsulfinyl group having a carbon number of 1 to 4, is more preferably an alkylsulfinyl group having a carbon number of 1 or 2, and is particularly preferably a methylsulfinyl group.

The alkylsulfonyl group having a carbon number of 1 to 6 of Rc may be a methylsulfonyl group, an ethylsulfonyl group, a propylsulfonyl group, an isopropylsulfonyl group, a butylsulfonyl group, an isobutylsulfonyl group, a sec-butylsulfonyl group, a tert-butylsulfonyl group, a pentylsulfonyl group, a hexylsulfonyl group, or the like, is preferably an alkylsulfonyl group having a carbon number of 1 to 4, is more preferably an alkylsulfonyl group having a carbon number of 1 or 2, and is particularly preferably a methylsulfonyl group.

The fluoroalkyl group having a carbon number of 1 to 6 of Rc may be a fluoromethyl group, a trifluoromethyl group, a fluoroethyl group, a pentafluoroethyl group, a heptafluoropropyl group, a nonafluorobutyl group, or the like, is preferably a fluoroalkyl group having a carbon number of 1 to 4, is more preferably a fluoroalkyl group having a carbon number of 1 or 2, and is particularly preferably a trifluoromethyl group.

The alkoxy group having a carbon number of 1 to 6 of Rc may be a methoxy group, an ethoxy group, a propoxy group, an isopropoxy group, a butoxy group, an isobutoxy group, a sec-butoxy group, a tert-butoxy group, a pentyloxy group, a hexyloxy group, or the like, is preferably an alkoxy group having a carbon number of 1 to 4, is more preferably an alkoxy group having a carbon number of 1 or 2, and is particularly preferably a methoxy group.

The thioalkyl group having a carbon number of 1 to 6 of Rc may be a methylthio group, an ethylthio group, a propylthio group, an isopropylthio group, a butylthio group, an isobutylthio group, a sec-butylthio group, a tert-butylthio group, a pentylthio group, a hexylthio group, or the like, is preferably a thioalkyl group having a carbon number of 1 to 4, is more preferably a thioalkyl group having a carbon number of 1 or 2, and is particularly preferably a methylthio group.

The N-alkylamino group having a carbon number of 1 to 6 of Rc may be an N-methylamino group, an N-ethylamino group, an N-propylamino group, an N-isopropylamino group, an N-butylamino group, an N-isobutylamino group, an N-sec-butylamino group, an N-tert-butylamino group, an N-pentylamino group, an N-hexylamino group, or the like, is preferably an N-alkylamino group having a carbon number of 1 to 4, more preferably an N-alkylamino group having a carbon number of 1 or 2, and particularly preferably an N-methylamino group.

The N,N-dialkylamino group having a carbon number of 2 to 12 of Rc may be an N,N-dimethylamino group, an N-methyl-N-ethylamino group, an N,N-diethylamino group, an N,N-dipropylamino group, an N,N-diisopropylamino group, an N,N-dibutylamino group, an N,N-diisobutylamino group, an N,N-dipentylamino group, an N,N-dihexylamino group, or the like, is preferably an N,N-dialkylamino group having a carbon number of 2 to 8, is more preferably an N,N-dialkylamino group having a carbon number of 2 to 4, and is particularly preferably an N,N-dimethylamino group.

The N-alkylsulfamoyl group having a carbon number of 1 to 6 of Rc may be an N-methylsulfamoyl group, an N-ethylsulfamoyl group, an N-propylsulfamoyl group, an N-isopropylsulfamoyl group, an N-butylsulfamoyl group, an N-isobutylsulfamoyl group, an N-sec-butylsulfamoyl group, an N-tert-butylsulfamoyl group, an N-pentylsulfamoyl group, an N-hexylsulfamoyl group, or the like, is preferably an N-alkylsulfamoyl group having a carbon number of 1 to 4, is more preferably an N-alkylsulfamoyl group having a carbon number of 1 or 2, and is particularly preferably an N-methylsulfamoyl group.

The N,N-dialkylsulfamoyl group having a carbon number of 2 to 12 of Rc may be an N,N-dimethylsulfamoyl group, an N-methyl-N-ethylsulfamoyl group, an N,N-diethylsulfamoyl group, an N,N-dipropylsulfamoyl group, an N,N-diisopropylsulfamoyl group, an N,N-dibutylsulfamoyl group, an N,N-diisobutylsulfamoyl group, an N,N-dipentylsulfamoyl group, an N,N-dihexylsulfamoyl group, or the like, is preferably an N,N-dialkylsulfamoyl group having a carbon number of 2 to 8, is more preferably an N,N-dialkylsulfamoyl group having a carbon number of 2 to 4, and is particularly preferably an N,N-dimethylsulfamoyl group.

Of the examples set forth above, Rc is preferably a halogen atom, a tert-butyl group, a methyl group, a cyano group, a nitro group, a carboxyl group, a methylsulfonyl group, a trifluoromethyl group, a methoxy group, a methylthio group, an N-methylamino group, an N,N-dimethylamino group, an N-methylsulfamoyl group, an N,N-dimethylsulfamoyl group, or a methylsulfinyl group.

In formulae (II-1) to (II-4), $E^1$ and $E^2$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number of 1 to 4. The alkyl group having a carbon number of 1 to 4 of $R^{11}$ and $R^{12}$ may be a methyl group, an ethyl group, an n-propyl group, an isopropyl group, a butyl group, an isobutyl group, a sec-butyl group, a tert-butyl group, or the like, is preferably an alkyl group having a carbon number of 1 or 2, and is more preferably a methyl group.

Moreover, it is preferable that $E^1$ and $E^2$ are each, independently of one another, -S-, -C(=O)-, -NH-, or $-N(CH_3)-$.

In formulae (II-1) to (II-4), $D^1$ and $D^2$ each represent, independently of one another, an optionally substituted aromatic hydrocarbon cyclic group or an optionally substituted aromatic heterocyclic group.

Specifically, the aromatic hydrocarbon cyclic group of $D^1$ and $D^2$ may be a phenyl group, a naphthyl group, an anthracenyl group, a phenanthrenyl group, a pyrenyl group, a fluorenyl group, or the like.

Of these examples, the aromatic hydrocarbon cyclic group is preferably a phenyl group or a naphthyl group.

The aromatic heterocyclic group of $D^1$ and $D^2$ may be a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, a benzothiazolyl group, a benzothienyl group, a benzotriazinyl group, a benzotriazolyl group, a benzopyrazolyl group, a benzopyranonyl group, a dihydropyranyl group, a tetrahydropyranyl group, a dihydrofuranyl group, a tetrahydrofuranyl group, or the like.

Of these examples, the aromatic heterocyclic group is preferably a furanyl group, a thienyl group, an oxazolyl group, a thiazolyl group, a benzothiazolyl group, a benzoxazolyl group, a 1-benzofuranyl group, a 2-benzofuranyl group,

a benzothienyl group, or a thiazolopyridyl group.

The aromatic hydrocarbon cyclic group and the aromatic heterocyclic group of $D^1$ and $D^2$ may be substituted with a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12.

Note that the aromatic hydrocarbon cyclic group and the aromatic heterocyclic group may have one or a plurality of substituents selected from the substituents set forth above. Moreover, in a case in which the group has a plurality of substituents, these substituents may be the same or different.

Examples of the halogen atom, the alkyl group having a carbon number of 1 to 6, the alkylsulfinyl group having a carbon number of 1 to 6, the alkylsulfonyl group having a carbon number of 1 to 6, the fluoroalkyl group having a carbon number of 1 to 6, the alkoxy group having a carbon number of 1 to 6, the thioalkyl group having a carbon number of 1 to 6, the N-alkylamino group having a carbon number of 1 to 6, the N,N-dialkylamino group having a carbon number of 2 to 12, the N-alkylsulfamoyl group having a carbon number of 1 to 6, and the N,N-dialkylsulfamoyl group having a carbon number of 2 to 12 that may be substituents of $D^1$ and $D^2$ and preferable examples thereof include the same specific examples and preferable examples as listed for the halogen atom, the alkyl group having a carbon number of 1 to 6, the alkylsulfinyl group having a carbon number of 1 to 6, the alkylsulfonyl group having a carbon number of 1 to 6, the fluoroalkyl group having a carbon number of 1 to 6, the alkoxy group having a carbon number of 1 to 6, the thioalkyl group having a carbon number of 1 to 6, the N-alkylamino group having a carbon number of 1 to 6, the N,N-dialkylamino group having a carbon number of 2 to 12, the N-alkylsulfamoyl group having a carbon number of 1 to 6, and the N,N-dialkylsulfamoyl group having a carbon number of 2 to 12 of Rc.

$D^1$ and $D^2$ are preferably each, independently of one another, any one of groups represented by the following formulae (III-1) to (III-8).

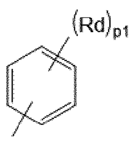 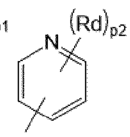 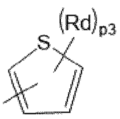 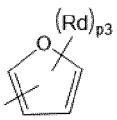 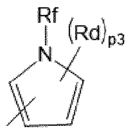 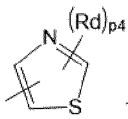 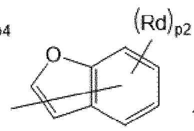 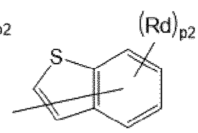

(III-1) (III-2) (III-3) (III-4) (III-5) (III-6) (III-7) (III-8)

In formulae (III-1) to (III-8), Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12. Moreover, p1 represents an integer of 0 to 5, p2 represents an integer of 0 to 4, p3 represents an integer of 0 to 3, and p4 represents an integer of 0 to 2. In particular, p1, p3, and p4 are preferably each 0 or 1, and p2 is preferably an integer of 0 to 3. Furthermore, Rf represents a hydrogen atom or a methyl group.

In a case in which more than one Rd is present in any of formulae (III-1) to (III-8) (i.e., in a case in which p1, p2, p3, or p4 is 2 or more), each Rd may be the same or different.

Examples of the halogen atom, the alkyl group having a carbon number of 1 to 6, the alkylsulfinyl group having a carbon number of 1 to 6, the alkylsulfonyl group having a carbon number of 1 to 6, the fluoroalkyl group having a carbon number of 1 to 6, the alkoxy group having a carbon number of 1 to 6, the thioalkyl group having a carbon number of 1 to 6, the N-alkylamino group having a carbon number of 1 to 6, the N,N-dialkylamino group having a carbon number of 2 to 12, the N-alkylsulfamoyl group having a carbon number of 1 to 6, and the N,N-dialkylsulfamoyl group having a carbon number of 2 to 12 of Rd and preferable examples thereof include the same specific examples and preferable examples as listed for the halogen atom, the alkyl group having a carbon number of 1 to 6, the alkylsulfinyl group having a carbon number of 1 to 6, the alkylsulfonyl group having a carbon number of 1 to 6, the fluoroalkyl group having a carbon number of 1 to 6, the alkoxy group having a carbon number of 1 to 6, the thioalkyl group having a carbon number of 1 to 6, the N-alkylamino group having a carbon number of 1 to 6, the N,N-dialkylamino group having a carbon number of 2 to 12, the N-alkylsulfamoyl group having a carbon number of 1 to 6, and the N,N-dialkylsulfamoyl group having a carbon number of 2 to 12 of Rc.

Rd is preferably a halogen atom, a methyl group, a cyano group, a nitro group, a carboxyl group, a trifluoromethyl group, a methoxy group, a methylthio group, an N,N-dimethylamino group, or an N-methylamino group.

A case in which $D^1$ and $D^2$ are each, independently of one another, a group represented by formula (III-1), (III-3), or (III-7) is particularly preferable in terms of cost and optical properties of the polymerizable compound (I).

In the previously mentioned formula (I), $Ar^1$ is more preferably any one of groups represented by the following formulae (IV-1) to (IV-5).

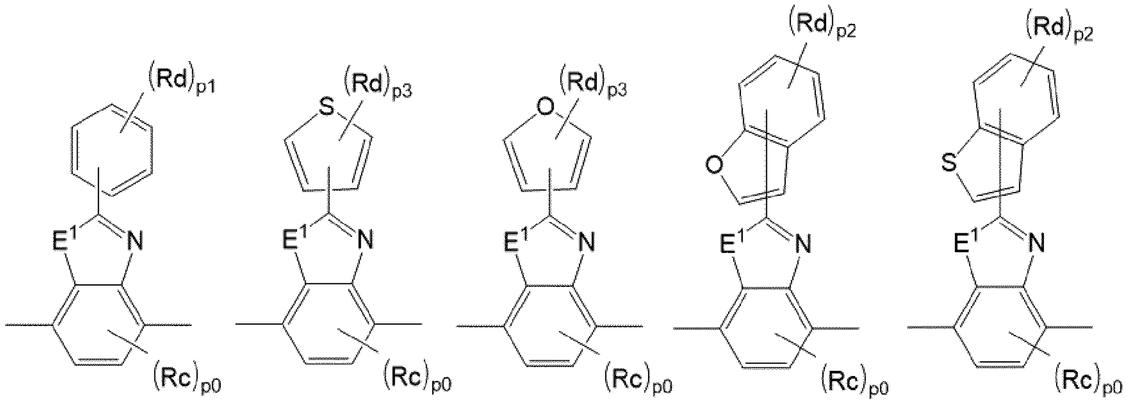

(IV-1) (IV-2) (IV-3) (IV-4) (IV-5)

In formulae (IV-1) to (IV-5), $E^1$, Rc, Rd, and p0 to p3 have the same meaning as previously described and preferable examples thereof are also the same as previously described.

Specific examples of $Ar^1$ are indicated by the following formulae (ar-1) to (ar-94).

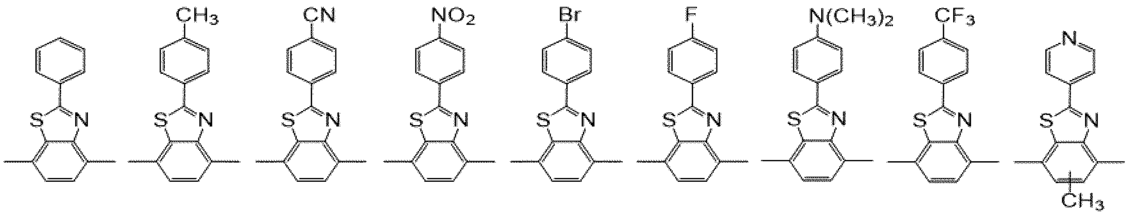

(ar-1) (ar-2) (ar-3) (ar-4) (ar-5) (ar-6) (ar-7) (ar-8) (ar-9)

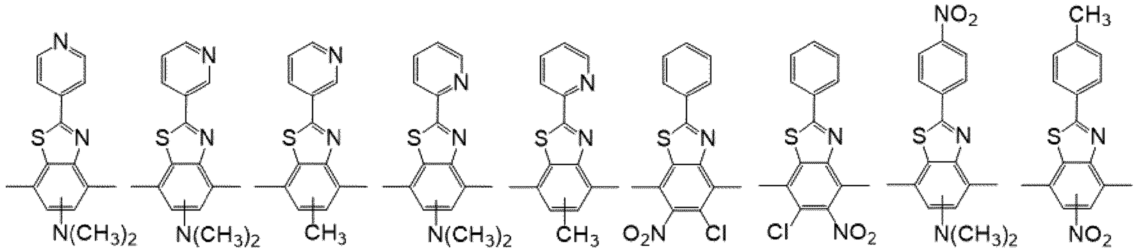

(ar-10) (ar-11) (ar-12) (ar-13) (ar-14) (ar-15) (ar-16) (ar-17) (ar-18)

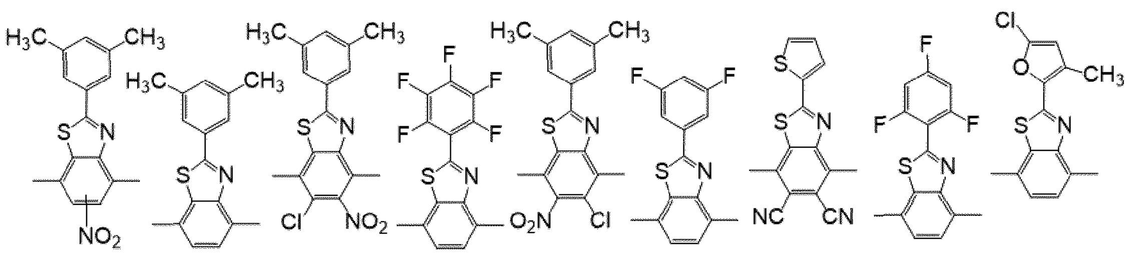

(ar-19) (ar-20) (ar-21) (ar-22) (ar-23) (ar-24) (ar-25) (ar-26) (ar-27)

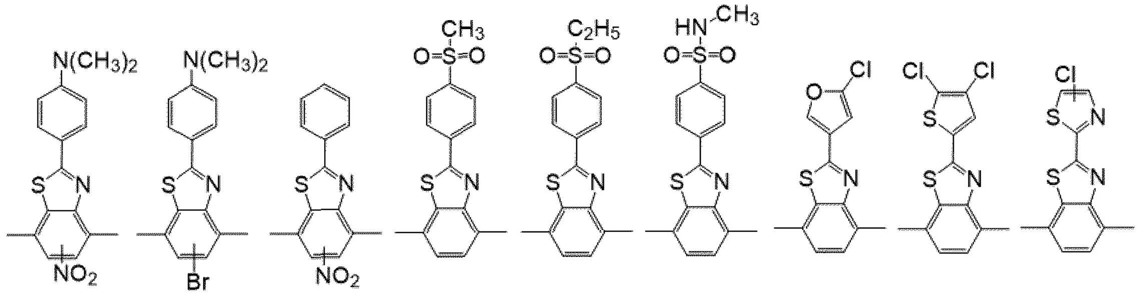

(ar-28) (ar-29) (ar-30) (ar-31) (ar-32) (ar-33) (ar-34) (ar-35) (ar-36)

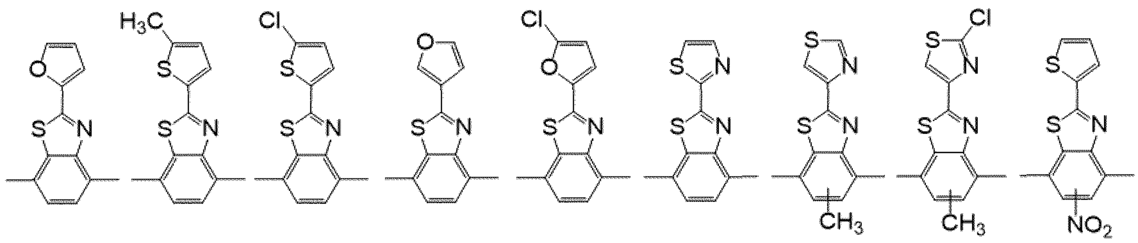

(ar-37) (ar-38) (ar-39) (ar-40) (ar-41) (ar-42) (ar-43) (ar-44) (ar-45)

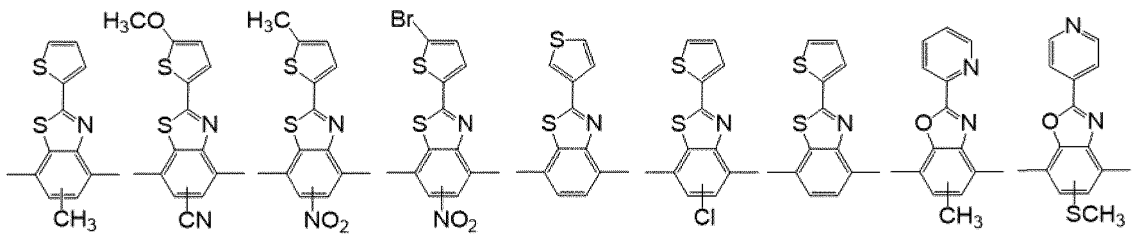

(ar-46) (ar-47) (ar-48) (ar-49) (ar-50) (ar-51) (ar-52) (ar-53) (ar-54)

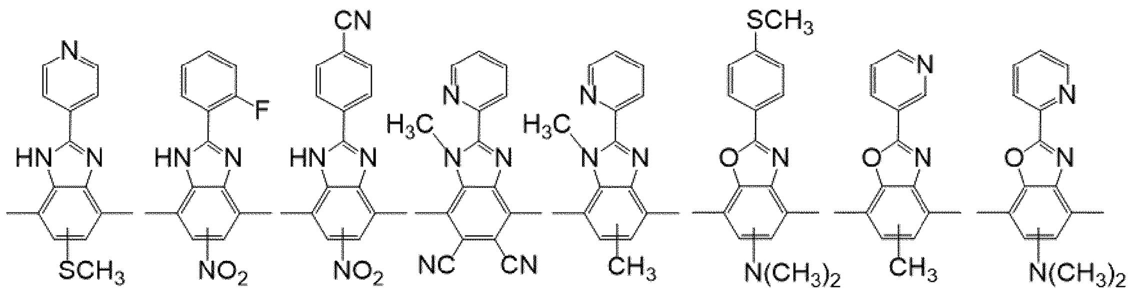

(ar-55) (ar-56) (ar-57) (ar-58) (ar-59) (ar-60) (ar-61) (ar-62)

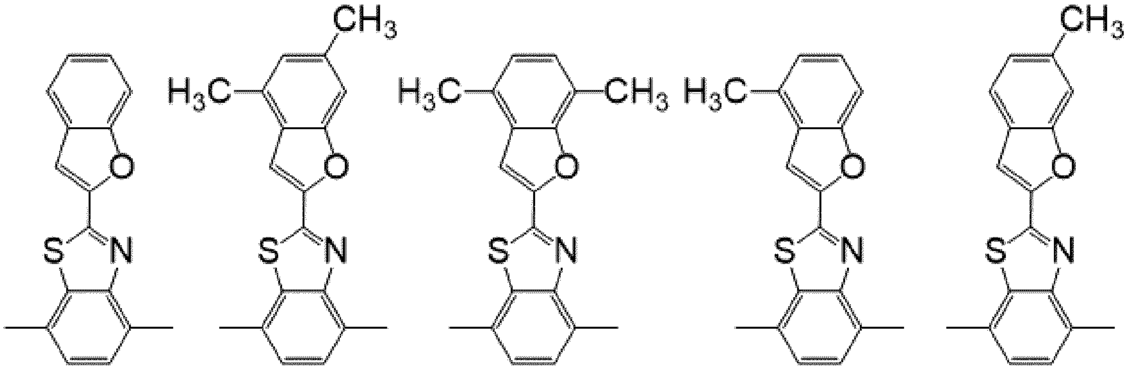

(ar-63) (ar-64) (ar-65) (ar-66) (ar-67)

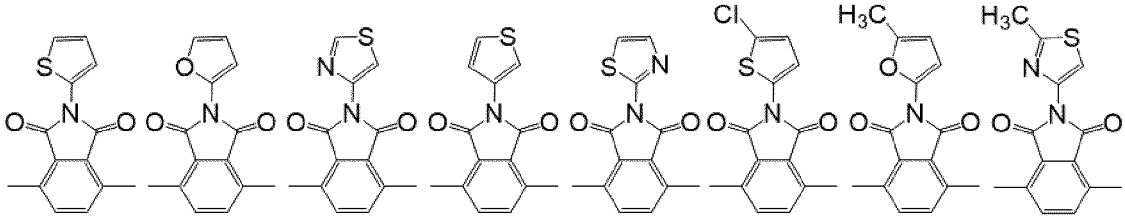

(ar-68) (ar-69) (ar-70) (ar-71) (ar-72) (ar-73) (ar-74) (ar-75)

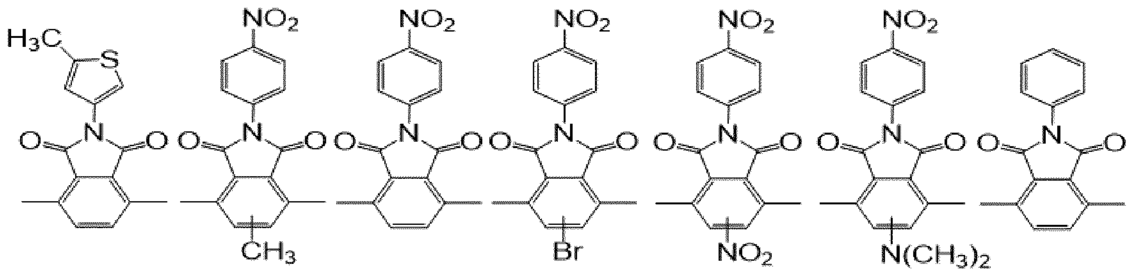

(ar-76) (ar-77) (ar-78) (ar-79) (ar-80) (ar-81) (ar-82)

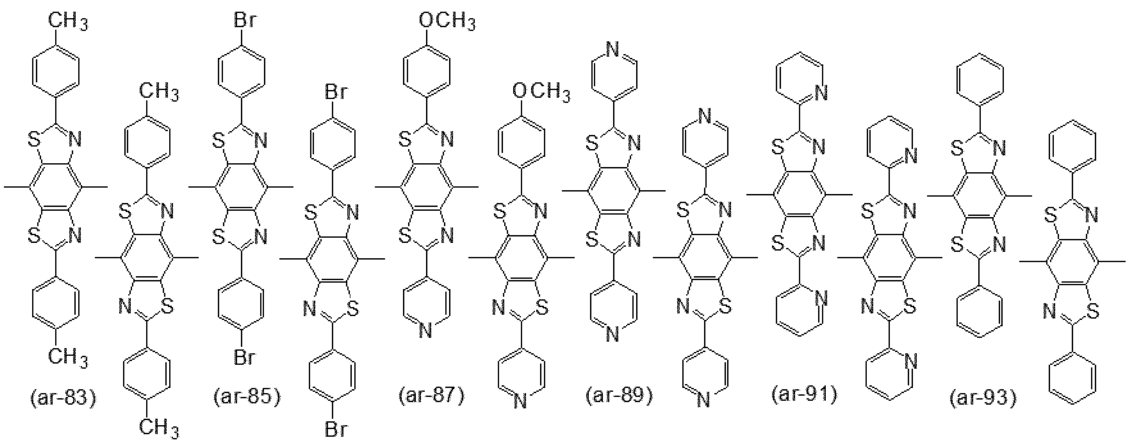

(ar-83) (ar-84) (ar-85) (ar-86) (ar-87) (ar-88) (ar-89) (ar-90) (ar-91) (ar-92) (ar-93) (ar-94)

In the previously mentioned formula (I), $Z^1$ and $Z^2$ are each, independently of one another, a single bond, -O-$CH_2$-, -$CH_2$-O-, -C(=O)-O-, -O-C(=O)-, -C(=O)-S-, -S-C(=O)-, -$NR^{13}$-C(=O)-, -C(=O)-$NR^{13}$-, -$CF_2$-O-, -O-$CF_2$-,

$-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-. $R^{13}$ is a hydrogen atom or an alkyl group having a carbon number of 1 to 6. The alkyl group having a carbon number of 1 to 6 of $R^{13}$ may, for example, be a methyl group, an ethyl group, a propyl group, an isopropyl group, or the like.

Of these examples, $Z^1$ is preferably -CO-O-. Moreover, $Z^2$ is preferably -O-CO-.

$A^1$ and $A^2$ are each, independently of one another, an optionally substituted alicyclic group or an optionally substituted aromatic group. Of these examples, $A^1$ and $A^2$ are preferably each an optionally substituted alicyclic group.

The optionally substituted alicyclic group is an unsubstituted divalent alicyclic group or a substituted divalent alicyclic group. Moreover, the divalent alicyclic group is a divalent aliphatic group having a cyclic structure and typically having a carbon number of 5 to 20.

Specific examples of the divalent alicyclic group of $A^1$ and $A^2$ include cycloalkanediyl groups having a carbon number of 5 to 20 such as cyclopentane-1,3-diyl, cyclohexane-1,4-diyl, cycloheptane-1,4-diyl, and cyclooctane-1,5-diyl; and bicycloalkanediyl groups having a carbon number of 5 to 20 such as decahydronaphthalene-1,5-diyl and decahydronaphthalene-2,6-diyl.

The optionally substituted aromatic group is an unsubstituted divalent aromatic group or a substituted divalent aromatic group. Moreover, the divalent aromatic group is a divalent aromatic group having an aromatic ring structure and typically having a carbon number of 2 to 20.

Specific examples of the divalent aromatic group of $A^1$ and $A^2$ include aromatic hydrocarbon cyclic groups having a carbon number of 6 to 20 such as a 1,4-phenylene group, a 1,4-naphthylene group, a 1,5-naphthylene group, a 2,6-naphthylene group, and a 4,4'-biphenylene group; and aromatic heterocyclic groups having a carbon number of 2 to 20 such as furan-2,5-diyl, thiophene-2,5-diyl, pyridine-2,5-diyl, and pyrazine-2,5-diyl.

Examples of possible substituents of the divalent alicyclic group and the divalent aromatic group of $A^1$ and $A^2$ include halogen atoms such as a fluorine atom, a chlorine atom, and a bromine atom; alkyl groups having a carbon number of 1 to 6 such as a methyl group and an ethyl group; alkoxy groups having a carbon number of 1 to 5 such as a methoxy group and an isopropoxy group; a nitro group; and a cyano group. The alicyclic group and the aromatic group may have one or more substituents selected from the substituents set forth above. Note that in a case in which the group has a plurality of substituents, these substituents may be the same or different.

In a case in which a and/or b is 1, $L^1$ and $L^2$ are each, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$. $R^{14}$ and $R^{15}$ are each, independently of one another, a hydrogen atom or an alkyl group having a carbon number of 1 to 6. Of these examples, $L^1$ and $L^2$ are preferably each, independently of one another, -O-, -CO-O-, or -O-CO-.

The alkyl group having a carbon number of 1 to 6 of $R^{14}$ and $R^{15}$ may, for example, be a methyl group, an ethyl group, a propyl group, an isopropyl group, or the like.

Moreover, in a case in which a and/or b is 1, $B^1$ and $B^2$ are each, independently of one another, an optionally substituted alicyclic group or an optionally substituted aromatic group. Of these examples, $B^1$ and $B^2$ are preferably each an optionally substituted aromatic group.

The optionally substituted alicyclic group is an unsubstituted divalent alicyclic group or a substituted divalent alicyclic group. Moreover, the divalent alicyclic group is a divalent aliphatic group having a cyclic structure and typically having a carbon number of 5 to 20.

Specific examples of the divalent alicyclic group of $B^1$ and $B^2$ include the same examples as listed for the divalent alicyclic group of $A^1$.

The optionally substituted aromatic group is an unsubstituted divalent aromatic group or a substituted divalent aromatic group. Moreover, the divalent aromatic group is a divalent aromatic group having an aromatic ring structure and typically having a carbon number of 2 to 20.

Specific examples of the divalent aromatic group of $B^1$ and $B^2$ include the same examples as listed for the divalent aromatic group of $A^1$.

Examples of possible substituents of the divalent alicyclic group and the divalent aromatic group of $B^1$ and $B^2$ include the same examples as listed as possible substituents of the divalent alicyclic group and the divalent aromatic group of $A^1$.

$Y^1$ and $Y^2$ are each, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$. $R^{14}$ and $R^{15}$ are each, independently of one another, a hydrogen atom or an alkyl group having a carbon number of 1 to 6. Of these examples, $Y^1$ and $Y^2$ are preferably each, independently of one another, -O-, -CO-O-, or -O-CO-.

The alkyl group having a carbon number of 1 to 6 of $R^{14}$ and $R^{15}$ may, for example, be a methyl group, an ethyl group, a propyl group, an isopropyl group, or the like.

$R^1$ to $R^4$ are each, independently of one another, a hydrogen atom, a methyl group, or a chlorine atom, and are preferably each a hydrogen atom or a methyl group. Note that it is more preferable that $R^1$ to $R^4$ are all the same,

and even more preferable that $R^1$ to $R^4$ are all hydrogen atoms.

The polymerizable compound (I) preferably has a structure in which the left and right sides are roughly symmetrical with $Ar^1$ as a center from a viewpoint of obtaining an optical film or the like having excellent reverse wavelength dispersion. Specifically, it is preferable that in the polymerizable compound (I), $R^1$, a, and c are the same as $R^4$, b, and d, respectively, and that $-Y^1-[B^1-L^1]_a-A^1-Z^1-(*)$ and $(*)-Z^2-A^2-[L^2-B^2]_b-Y^2-$ have symmetrical structures with sides (*) bonded to $Ar^1$ as the center of symmetry.

Note that "symmetrical structures with (*) as the center of symmetry" refers to structures such as -CO-O-(*) and (*)-O-CO-, -O-(*) and (*)-O-, or -O-CO-(*) and (*)-CO-O-, for example.

The polymerizable compound (I) can be synthesized through a combination of known synthetic reactions. Specifically, the polymerizable compound (I) can be synthesized with reference to methods described in various documents (for example, March's Advanced Organic Chemistry (Wiley); and Sandler and Karo, "Syntheses of Organic Compounds Classified by Functional Group", joint translation by Naoki INAMOTO (Hirokawa Publishing Company)) and JP 2010-031223 A.

(2) Mixture containing polymerizable compound

The presently disclosed mixture is a mixture that contains the polymerizable compound (I) and a polymerizable compound indicated by the following formula (V) (polymerizable compound (V)) and that can be used in production of a polymerizable liquid crystal composition and a polymer described further below, for example.

The mass ratio of the polymerizable compound (I) relative to the polymerizable compound (V) (polymerizable compound (I):polymerizable compound (V)) in the mixture is preferably 1:1,000 to 20:100, and more preferably 1:100 to 20:100 from a viewpoint of widening the process margin in formation of an optical film or the like using the mixture, or using a polymerizable liquid crystal composition prepared using the mixture, while also enhancing reverse wavelength dispersion of the obtained optical film.

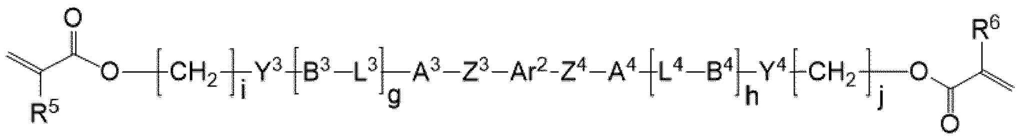

(V)

Through use of a mixture containing the polymerizable compound (I) and the polymerizable compound (V), it is possible to produce, at low cost, an optical film or the like that can maintain a liquid crystal phase over a long period with higher stability, has a wide process margin, has a practical low melting point, has excellent solubility in general purpose solvents, and that can perform uniform polarized light conversion over a wide wavelength region.

Although the reason for this is not clear, it is presumed that as a result of the polymerizable compound (I) having a structure indicated by $-(CH_2CHR^2COO)_e-$ and/or $-(OCOCHR^3CH_2)_f-$, it is possible to ensure optical properties (particularly reverse wavelength dispersion) while forming a liquid crystal layer that readily adopts a liquid crystal phase at lower temperature (i.e., readily adopts a supercooled state at room temperature) and obtaining an optical film or the like having a polymer as a constituent material using a mixture of the polymerizable compound (I) and the polymerizable compound (V), as compared to a case in which only the polymerizable compound (V) is used.

In formula (V), g and h are each, independently of one another, 0 or 1, and preferably 1. Moreover, i and j are each, independently of one another, an integer of 1 to 20, preferably an integer of 2 to 12, and more preferably an integer of 4 to 8.

$Ar^2$ is any one of groups represented by the following formulae (VI-1) to (VI-4).

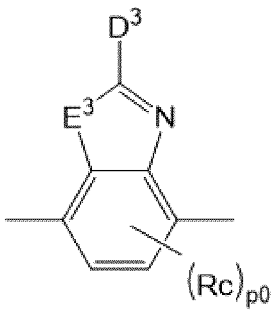
(VI-1)

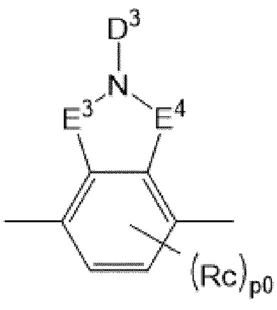
(VI-2)

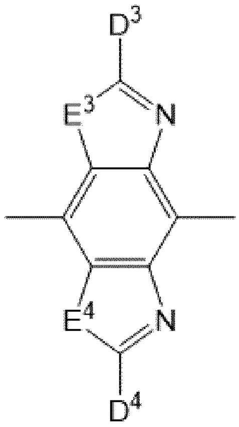
(VI-3)

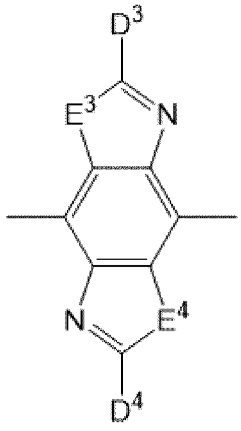
(VI-4)

In formulae (VI-1) to (VI-4), Rc and p0 have the same meaning as previously described and preferable examples thereof are also the same as previously described.

In formulae (VI-1) to (VI-4), $E^3$ and $E^4$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number of 1 to 4. The alkyl group having a carbon number of 1 to 4 of $R^{11}$ and $R^{12}$ may be a methyl group, an ethyl group, an n-propyl group, an isopropyl group, a butyl group, an isobutyl group, a sec-butyl group, a tert-butyl group, or the like, is preferably an alkyl group having a carbon number of 1 or 2, and is more preferably a methyl group.

Moreover, $E^3$ and $E^4$ are preferably each, independently of one another, -S-, -C(=O)-, -NH-, or $-N(CH_3)-$.

In formulae (VI-1) to (VI-4), $D^3$ and $D^4$ each represent, independently of one another, an optionally substituted aromatic hydrocarbon cyclic group or an optionally substituted aromatic heterocyclic group.

Examples of the aromatic hydrocarbon group and the aromatic heterocyclic group of $D^3$ and $D^4$ and possible substituents thereof include the same examples as listed for the aromatic hydrocarbon group and the aromatic heterocyclic group of $D^1$ and $D^2$ and possible substituents thereof.

$D^3$ and $D^4$ are preferably each, independently of one another, any one of groups represented by the previously shown formulae (III-1) to (III-8), and are particularly preferably each a group represented by formula (III-1), (III-3), or (III-7) in terms of cost and optical properties of the polymerizable compound (V).

In the previously mentioned formula (V), $Ar^2$ is more preferably any one of groups represented by the following formulae (VII-1) to (VII-5).

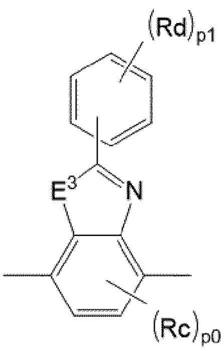
(VII-1)

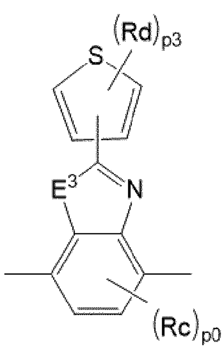
(VII-2)

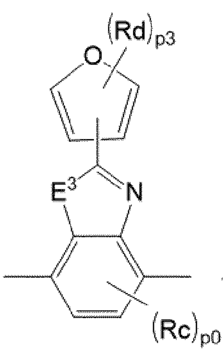
(VII-3)

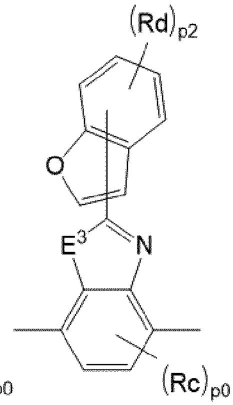
(VII-4)

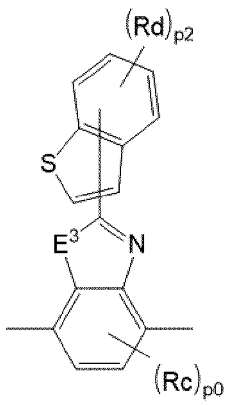
(VII-5)

In formulae (VII-1) to (VII-5), $E^3$, Rc, Rd, and p0 to p3 have the same meaning as previously described and preferable examples thereof are also the same as previously described.

Specific examples of $Ar^2$ include groups represented by the previously shown formulae (ar-1) to (ar-94).

In the previously mentioned formula (V), $Z^3$ and $Z^4$ are each, independently of one another, a single bond, $-O-CH_2-$, $-CH_2-O-$, -C(=O)-O-, -O-C(=O)-, -C(=O)-S-, -S-C(=O)-, $-NR^{13}-C(=O)-$, $-C(=O)-NR^{13}-$, $-CF_2-O-$, $-O-CF_2-$,

$-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-. $R^{13}$ is a hydrogen atom or an alkyl group having a carbon number of 1 to 6. The alkyl group having a carbon number of 1 to 6 of $R^{13}$ may, for example, be a methyl group, an ethyl group, a propyl group, an isopropyl group, or the like.

Of these examples, $Z^3$ is preferably -CO-O-. Moreover, $Z^4$ is preferably -O-CO-.

$A^3$ and $A^4$ are each, independently of one another, an optionally substituted alicyclic group or an optionally substituted aromatic group. Of these examples, $A^3$ and $A^4$ are preferably each an optionally substituted alicyclic group.

Examples of the optionally substituted alicyclic group and the optionally substituted aromatic group of $A^3$ and $A^4$ include the same as for the optionally substituted alicyclic group and the optionally substituted aromatic group of $A^1$ and $A^2$ in the polymerizable compound (I).

In a case in which g and/or h is 1, $L^3$ and $L^4$ are each, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$. $R^{14}$ and $R^{15}$ are each, independently of one another, a hydrogen atom or an alkyl group having a carbon number of 1 to 6. Of these examples, $L^3$ and $L^4$ are preferably each, independently of one another, -O-, -CO-O-, or -O-CO-.

The alkyl group having a carbon number of 1 to 6 of $R^{14}$ and $R^{15}$ may, for example, be a methyl group, an ethyl group, a propyl group, an isopropyl group, or the like.

Moreover, in a case in which g and/or h is 1, $B^3$ and $B^4$ are each, independently of one another, an optionally substituted alicyclic group or an optionally substituted aromatic group. Of these examples, $B^3$ and $B^4$ are preferably each an optionally substituted aromatic group.

Examples of the optionally substituted alicyclic group and the optionally substituted aromatic group of $B^3$ and $B^4$ include the same as for the optionally substituted alicyclic group and the optionally substituted aromatic group of $B^1$ and $B^2$ in the polymerizable compound (I).

$Y^3$ and $Y^4$ are each, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$. $R^{14}$ and $R^{15}$ are each, independently of one another, a hydrogen atom or an alkyl group having a carbon number of 1 to 6. Of these examples, $Y^3$ and $Y^4$ are preferably each, independently of one another, -O-, -CO-O-, or -O-CO-.

The alkyl group having a carbon number of 1 to 6 of $R^{14}$ and $R^{15}$ may, for example, be a methyl group, an ethyl group, a propyl group, an isopropyl group, or the like.

$R^5$ and $R^6$ are each, independently of one another, a hydrogen atom, a methyl group, or a chlorine atom, and are preferably each a hydrogen atom or a methyl group. Note that it is more preferable that $R^5$ is the same as $R^6$, and even more preferable that $R^5$ and $R^6$ are both hydrogen atoms.

The polymerizable compound (V) preferably has a structure in which the left and right sides are symmetrical with $Ar^2$ as a center from a viewpoint of obtaining an optical film or the like having excellent reverse wavelength dispersion. Specifically, it is preferable that in the polymerizable compound (V), $R^5$, g, and i are the same as $R^6$, h, and j, respectively, and that $-Y^3-[B^3-L^3]_g-A^3-Z^3-(*)$ and $(*)-Z^4-A^4-[L^4-B^4]_h-Y^4-$ have symmetrical structures with sides (*) bonded to $Ar^2$ as the center of symmetry. Note that "symmetrical structures with (*) as the center of symmetry" refers to structures such as -CO-O-(*) and (*)-O-CO-, -O-(*) and (*)-O-, or -O-CO-(*) and (*)-CO-O-, for example.

The polymerizable compound (V) can be synthesized through a combination of known synthetic reactions. Specifically, the polymerizable compound (V) can be synthesized with reference to methods described in various documents (for example, March's Advanced Organic Chemistry (Wiley); and Sandler and Karo, "Syntheses of Organic Compounds Classified by Functional Group", joint translation by Naoki INAMOTO (Hirokawa Publishing Company)) and JP 2010-031223 A.

From a viewpoint of enhancing reverse wavelength dispersion of an optical film or the like, it is preferable that $Ar^1$, $Z^1$, $Z^2$, $A^1$, $A^2$, $B^1$, $B^2$, $Y^1$, $Y^2$, $L^1$, $L^2$, $R^1$, $R^4$, and a to d of the polymerizable compound (I) in the presently disclosed mixture are the same as $Ar^2$, $Z^3$, $Z^4$, $A^3$, $A^4$, $B^3$, $B^4$, $Y^3$, $Y^4$, $L^3$, $L^4$, $R^5$, $R^6$, and g to j, respectively, of the polymerizable compound (V) in the presently disclosed mixture. However, this is not a specific limitation on the presently disclosed mixture.

In other words, the polymerizable compound (V) preferably has the same structure as the polymerizable compound (I) with the exception of not including $-(CH_2CHR^2COO)_e-$ between $CH_2CR^1COO-$ and $-(CH_2)_c-$ and not including $-(OCOCHR^3CH_2)_f-$ between $-OCOCR^4CH_2$ and $-(CH_2)_d-$.

The mixture can be obtained by, for example, mixing the polymerizable compound (I) and the polymerizable compound (V) in a desired ratio.

(3) Polymerizable liquid crystal composition

The presently disclosed polymerizable liquid crystal composition contains the mixture containing the polymerizable compounds set forth above (mixture containing polymerizable compound (I) and polymerizable compound (V))

and a polymerization initiator.

The presently disclosed polymerizable liquid crystal composition is useful as a production raw material for the presently disclosed polymer, optical film, and optically anisotropic body as described further below. Through use of the presently disclosed polymerizable liquid crystal composition, it is possible to produce, at low cost, an optical film or the like that can maintain a liquid crystal phase over a long period with higher stability, has a wide process margin, has a practical low melting point, has excellent solubility in general purpose solvents, and can perform uniform polarized light conversion over a wide wavelength region.

The polymerization initiator is included from a viewpoint of more efficiently carrying out a polymerization reaction of the polymerizable compounds contained in the polymerizable liquid crystal composition.

Examples of polymerization initiators that may be used include radical polymerization initiators, anionic polymerization initiators, and cationic polymerization initiators.

Although both thermal radical generators, which are compounds that generate active species that can initiate polymerization of the polymerizable compounds upon heating, and photo-radical generators, which are compounds that generate active species that can initiate polymerization of the polymerizable compounds upon exposure to exposure light such as visible light rays, ultraviolet rays (i-line, etc.), far ultraviolet rays, an electron beam, or X-rays, can be used as the radical polymerization initiator, use of a photo-radical generator is preferable.

Examples of photo-radical generators that may be used include acetophenone compounds, biimidazole compounds, triazine compounds, O-acyl oxime compounds, onium salt compounds, benzoin compounds, benzophenone compounds, $\alpha$-diketone compounds, polynuclear quinone compounds, xanthone compounds, diazo compounds, and imide sulfonate compounds. These compounds are components that generate active radicals, active acid, or both active radicals and active acid upon photoexposure. One photo-radical generator may be used individually, or two or more photo-radical generators may be used in combination.

Specific examples of acetophenone compounds that may be used include 2-hydroxy-2-methyl-1-phenylpropan-1-one, 2-methyl-1-[4-(methylthio)phenyl]-2-morpholinopropan-1-one, 2-benzyl-2-dimethylamino-1-(4-morpholinophenyl)butan-1-one, 1-hydroxycyclohexyl phenyl ketone, 2,2-dimethoxy-1,2-diphenylethan-1-one, 1,2-octanedione, and 2-benzyl-2-dimethylamino-4'-morpholinobutyrophenone.

Specific examples of biimidazole compounds that may be used include 2,2'-bis(2-chlorophenyl)-4,4',5,5'-tetrakis(4-ethoxycarbonylphenyl)-1,2'-biim idazole, 2,2'-bis(2-bromophenyl)-4,4',5,5'-tetrakis(4-ethoxycarbonylphenyl)-1,2'-biim idazole, 2,2'-bis(2-chlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2-bromophenyl)-4,4',5,5'-tetraphenyl-1,2'-biimidazole, 2,2'-bis(2,4-dibromophenyl)-4,4',5,5'-tetraphenyl-1,2'-biimidazole, and 2,2'-bis(2,4,6-tribromophenyl)-4,4',5,5'-tetraphenyl-1,2'-biimidazole.

In a situation in which a biimidazole compound is used as a photoinitiator (photo-radical generator) in the present disclosure, it is preferable that a hydrogen donor is used in combination therewith in terms that sensitivity can be further enhanced.

The term "hydrogen donor" refers to a compound that can donate a hydrogen atom to a radical generated from the biimidazole compound upon photoexposure. The hydrogen donor is preferably a mercaptan compound, an amine compound, or the like such as defined below.

Examples of mercaptan compounds that may be used include 2-mercaptobenzothiazole, 2-mercaptobenzoxazole, 2-mercaptobenzimidazole, 2,5-dimercapto-1,3,4-thiadiazole, and 2-mercapto-2,5-dimethylaminopyridine. Examples of amine compounds that may be used include 4,4'-bis(dimethylamino)benzophenone, 4,4'-bis(diethylamino)benzophenone, 4-diethylaminoacetophenone, 4-dimethylaminopropiophenone, ethyl 4-dimethylaminobenzoate, 4-dimethylaminobenzoic acid, and 4-dimethylaminobenzonitrile.

Examples of triazine compounds that may be used include halomethyl group-containing triazine compounds such as 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-(furan-2-yl)ethenyl]-4,6-bis(trichloromethyl)-s-triazine, 2-[2-(4-diethylamino-2-methylphenyl)ethenyl]-4,6-bis(trichloromethyl)-s-tria zine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, and 2-(4-n-butoxyphenyl)-4,6-bis(trichloromethyl)-s-triazine.

Specific examples of O-acyl oxime compounds that may be used include 1-[4-(phenylthio)phenyl]-heptan-1,2-dione 2-(O-benzoyloxime), 1-[4-(phenylthio)phenyl]-octan-1,2-dione 2-(O-benzoyloxime), 1-[4-(benzoyl)phenyl]-octan-1,2-dione 2-(O-benzoyloxime), 1-[9-ethyl-6-(2-methylbenzoyl)-9H-carbazol-3-yl]-ethanone 1-(O-acetyloxime), 1-[9-ethyl-6-(3-methylbenzoyl)-9H-carbazol-3-yl]ethanone 1-(O-acetyloxime), 1-(9-ethyl-6-benzoyl-9H-carbazol-3-yl)-ethanone 1-(O-acetyloxime), ethanone-1-[9-ethyl-6-(2-methyl-4-tetrahydrofuranylbenzoyl)-9H-carbazol-3-yl]-1-(O-acetyloxime), ethanone-1-[9-ethyl-6-(2-methyl-4-tetrahydropyranylbenzoyl)-9H-carbazol-3-yl]-1-(O-acetyloxime), ethanone-1-[9-ethyl-6-(2-methyl-5-tetrahydrofuranylbenzoyl)-9H-carbazol-3-yl]-1-(O-acetyloxime), ethanone-1-[9-ethyl-6-(2-methyl-5-tetrahydropyranylbenzoyl)-9H-carbazol-3-yl]-1-(O-acetyloxime), ethanone-1-[9-ethyl-6-{2-methyl-4-(2,2-dimethyl-1,3-dioxolanyl)benzoyl}-9 H-carbazol-3-yl]-1-(O-acetyloxime), ethanone-1-[9-ethyl-6-(2-methyl-4-tetrahydrofuranylmethoxybenzoyl)-9H-car bazol-3-yl]-1-(O-acetyloxime), ethanone-1-[9-ethyl-6-(2-methyl-4-tetrahydropyranylmethoxybenzoyl)-9H-ca rbazol-3-yl]-1-(O-acetyloxime), ethanone-1-[9-ethyl-6-(2-methyl-5-tetrahydrofuranylmethoxybenzoyl)-9H-car bazol-3-yl]-1-(O-acetyloxime), ethanone-1-[9-ethyl-6-(2-methylbenzoyl)-9H-carbazol-3-yl]-1-(O-acetyloxim e), ethanone-1-[9-ethyl-6-(2-methyl-5-tetrahydropyranylmethoxybenzoyl)-9H-ca rbazol-3-yl]-1-(O-acetyloxime), and ethanone-1-[9-ethyl-6-{2-methyl-4-(2,2-dimethyl-1,3-dioxolanyl)methoxyben zoyl}-9H-carbazol-3-yl]-1-(O-acetyloxime).

A commercially available product may be used as a photo-radical generator. Specific examples include Irgacure 907 (product name), Irgacure 184 (product name), Irgacure 369 (product name), Irgacure 651 (product name), Irgacure 819 (product name), Irgacure 907 (product name), and Irgacure OXE02 (product name) produced by BASF, and ADEKA ARKLS N1919T (product name) produced by ADEKA Corporation.

Examples of anionic polymerization initiators that may be used include alkyllithium compounds; monolithium salts and monosodium salts of biphenyl, naphthalene, pyrene, and the like; and polyfunctional initiators such as dilithium salts and trilithium salts.

Examples of cationic polymerization initiators that may be used include proton acids such as sulfuric acid, phosphoric acid, perchloric acid, and trifluoromethanesulfonic acid; Lewis acids such as boron trifluoride, aluminum chloride, titanium tetrachloride, and tin tetrachloride; aromatic onium salts; and combinations of an aromatic onium salt and a reducing agent.

One of these polymerization initiators may be used individually, or two or more of these polymerization initiators may be used in combination.

The proportion in which the polymerization initiator is compounded in the presently disclosed polymerizable liquid crystal composition is normally 0.1 parts by mass to 30 parts by mass, and preferably 0.5 parts by mass to 10 parts by mass per 100 parts by mass of the previously described polymerizable compound-containing mixture.

Moreover, a surfactant is preferably compounded in the presently disclosed polymerizable liquid crystal composition in order to adjust surface tension. Although no specific limitations are placed on the surfactant, a non-ionic surfactant is normally preferable. The non-ionic surfactant may be a commercially available product. For example, a non-ionic surfactant that is an oligomer having a molecular weight of the order of thousands may be used. An example thereof is Ftergent 208G (product name) produced by Neos Company Limited.

The proportion in which the surfactant is compounded in the presently disclosed polymerizable liquid crystal composition is normally 0.01 parts by mass to 10 parts by mass, and preferably 0.1 parts by mass to 2 parts by mass per 100 parts by mass of all polymerizable compound.

Besides the polymerizable compound-containing mixture, the polymerization initiator, and the surfactant, the presently disclosed polymerizable liquid crystal composition may further contain other components to the extent that the effects disclosed herein are not affected. Examples of these other components include metals, metal complexes, dyes, pigments, fluorescent materials, phosphorescent materials, leveling agents, thixotropic agents, gelling agents, polysaccharides, ultraviolet absorbers, infrared absorbers, antioxidants, ion exchange resins, and metal oxides such as titanium oxide.

Moreover, other copolymerizable monomers may be used as other components. Specific examples include, but are not specifically limited to, 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4'-difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)biphenyl, 4-acryloyloxy-4'-propylcyclohexylphenyl, 4-methacryloyl-4'-butylbicyclohexyl, 4-acryloyl-4'-amyltolan, 4-acryloyl-4'-(3,4-difluorophenyl)bicyclohexyl, 4-amylphenyl 4-(2-acryloyloxyethyl)benzoate, 4-(4'-propylcyclohexyl)phenyl 4-(2-acryloyloxyethyl)benzoate, LC-242 (product name) produced by BASF, trans-1,4-bis[4-[6-(acryloyloxy)hexyloxy]phenyl]cyclohexanedicarboxylate, and other copolymerizable monomers such as compounds disclosed in JP 2007-002208 A, JP 2009-173893 A, JP 2009-274984 A, JP 2010-030979 A, JP 2010-031223 A, JP 2011-006360 A, and JP 2010-24438 A.

The proportion in which these other components are compounded is normally 0.1 parts by mass to 20 parts by mass per 100 parts by mass of all polymerizable compound.

The presently disclosed polymerizable liquid crystal composition can normally be produced by mixing/dissolving specific amounts of the polymerizable compound-containing mixture, the polymerization initiator, other components compounded as desired, and so forth in an appropriate organic solvent.

In this case, the polymerizable compound (I) and the polymerizable compound (V) used as the mixture may be added in a premixed state or may be added separately.

Examples of organic solvents that may be used include ketones such as cyclopentanone, cyclohexanone, and methyl ethyl ketone; acetic acid esters such as butyl acetate and amyl acetate; halogenated hydrocarbons such as chloroform, dichloromethane, and dichloroethane; and ethers such as 1,4-dioxane, cyclopentyl methyl ether, tetrahydrofuran, tetrahydropyran, and 1,3-dioxolane.

(4) Polymer

The presently disclosed polymer is obtained through polymerization of the previously described polymerizable compound-containing mixture (mixture containing polymerizable compound (I) and polymerizable compound (V)) or the previously described polymerizable liquid crystal composition.

Herein, the term "polymerization" is used to refer to a chemical reaction in a broad sense that is inclusive of a normal polymerization reaction and also a crosslinking reaction.

The presently disclosed polymer normally includes a monomer unit shown below that is derived from the polymerizable compound (I) (repeating unit (I)') and a monomer unit shown below that is derived from the polymerizable compound (V) (repeating unit (V)').

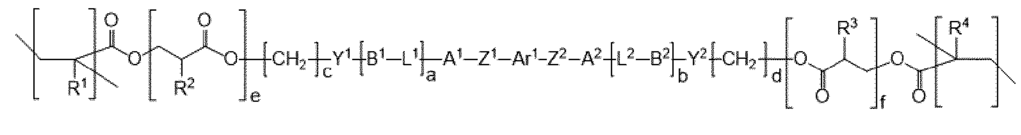

(I)'

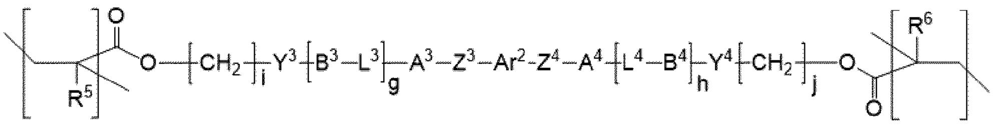

(V)'

[In formulae (I)' and (V)', $Ar^1$, $Ar^2$, $Z^1$ to $Z^4$, $A^1$ to $A^4$, $B^1$ to $B^4$, $Y^1$ to $Y^4$, $L^1$ to $L^4$, $R^1$ to $R^6$, and a to j have the same meaning as previously described.]

The presently disclosed polymer can favorably be used as a constituent material of an optical film or the like as a result of being produced using the mixture containing the polymerizable compound (I) and the polymerizable compound (V).

The presently disclosed polymer may be used in any form depending on the application, such as in the form of a film, a powder, or a layer of aggregated powder, without any specific limitations.

Specifically, a film of the polymer can favorably be used as a constituent material of the subsequently described optical film and optically anisotropic body, a powder of the polymer can be used for a paint, an anti-counterfeiting article, a security article, or the like, and a layer formed from a powder of the polymer can favorably be used as a constituent material of an optically anisotropic body.

The presently disclosed polymer can be suitably produced by ($\alpha$) carrying out a polymerization reaction of the polymerizable compound-containing mixture or the polymerizable liquid crystal composition in an appropriate organic solvent, subsequently isolating the target polymer, dissolving the obtained polymer in an appropriate organic solvent to prepare a solution, applying the solution onto an appropriate substrate to obtain an applied film, drying the applied film, and subsequently performing heating as desired, or ($\beta$) dissolving the polymerizable compound-containing mixture or the polymerizable liquid crystal composition in an organic solvent, applying the resultant solution onto a substrate by a commonly known application method, removing the solvent, and then carrying out a polymerization reaction through heating or irradiation with active energy rays.

The organic solvent used in the polymerization reaction in method ($\alpha$) is not specifically limited so long as it is an inert organic solvent. Examples include aromatic hydrocarbons such as toluene, xylene, and mesitylene; ketones such as cyclohexanone, cyclopentanone, and methyl ethyl ketone; acetic acid esters such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Of these organic solvents, those having a boiling point of 60°C to 250°C are preferable, and those having a boiling point of 60°C to 150°C are more preferable from a viewpoint of having excellent handleability.

Examples of the organic solvent in which the isolated polymer is dissolved in method ($\alpha$) and the organic solvent used in method ($\beta$) include ketone solvents such as acetone, methyl ethyl ketone, methyl isobutyl ketone, cyclopentanone, and cyclohexanone; ester solvents such as butyl acetate and amyl acetate; halogenated hydrocarbon solvents such as dichloromethane, chloroform, and dichloroethane; ether solvents such as tetrahydrofuran, tetrahydropyran, 1,2-dimethoxyethane, 1,4-dioxane, cyclopentyl methyl ether, and 1,3-dioxolane; and polar aprotic solvents such as N,N-dimethylformamide, N,N-dimethylacetamide, dimethyl sulfoxide, $\gamma$-butyrolactone, and N-methylpyrrolidone. Of these organic solvents, those having a boiling point of 60°C to 200°C are preferable in terms of ease of handling. These solvents may be used individually or as a combination of two or more types.

The substrate used in methods (α) and (β) may be made from a commonly known and typically used organic or inorganic material. Examples of organic materials that may be used include polycycloolefin (for example, ZEONEX® and ZEONOR® (ZEONEX and ZEONOR are registered trademarks in Japan, other countries, or both; produced by ZEON Corporation), ARTON® (ARTON is a registered trademark in Japan, other countries, or both; produced by JSR Corporation), and APEL® (APEL is a registered trademark in Japan, other countries, or both; produced by Mitsui Chemicals, Inc.)), polyethylene terephthalate, polycarbonate, polyimide, polyamide, polymethyl methacrylate, polystyrene, polyvinyl chloride, polytetrafluoroethylene, cellulose, cellulose triacetate, and polyethersulfone. Examples of inorganic materials that may be used include silicon, glass, and calcite.
The substrate that is used may be a single-layer substrate or a laminate.
The substrate is preferably a substrate formed from an organic material, and is more preferably a resin film obtained by shaping an organic material into the form of a film.
Examples of substrates that may be used also include substrates that can be used in preparation of the subsequently described optically anisotropic body.
Commonly known methods can be used as the method by which the solution of the polymer is applied onto the substrate in method (α) and the method by which the solution for polymerization reaction is applied onto the substrate in method (β). Specific examples of methods that may be used include curtain coating, extrusion coating, roll coating, spin coating, dip coating, bar coating, spray coating, slide coating, print coating, gravure coating, die coating, and cap coating.
The method of drying or solvent removal in methods (α) and (β) may be natural drying, heated drying, drying under reduced pressure, heated drying under reduced pressure, or the like.
The method by which polymerization of the mixture or polymerizable liquid crystal composition is carried out may, for example, be thermopolymerization or polymerization through irradiation with active energy rays. Polymerization through irradiation with active energy rays is preferable in terms that the reaction proceeds at room temperature without the need for heating. In particular, irradiation with light such as ultraviolet light is preferable due to the ease of operation.
The temperature during photoirradiation is preferably 30°C or lower. The photoirradiation intensity is normally within a range of 1 $W/m^2$ to 10 $kW/m^2$, and preferably within a range of 5 $W/m^2$ to 2 $kW/m^2$.
The polymer obtained as set forth above may be transferred from the substrate for use, may be peeled from the substrate and then used alone, or may be used as a constituent material or the like of an optical film or the like without being peeled from the substrate.
Moreover, polymer that is peeled from the substrate may be used after being pulverized by a known method to obtain a powder.
The number-average molecular weight of the presently disclosed polymer obtained as set forth above is preferably 500 to 500,000, and more preferably 5,000 to 300,000. A number-average molecular weight within any of the ranges set forth above is desirable because this provides high hardness and excellent handleability. The number-average molecular weight of the polymer can be measured by gel permeation chromatography (GPC) using monodisperse polystyrene as a standard sample and tetrahydrofuran as an eluent.
Through use of the presently disclosed polymer, it is possible to obtain, at low cost, an optical film or the like that can maintain a liquid crystal phase over a long period with higher stability, has a wide process margin, has a practical low melting point, has excellent solubility in general purpose solvents, and can perform uniform polarized light conversion over a wide wavelength region and display satisfactory performance.

(5) Optical film

The presently disclosed optical film includes a layer that is formed using the presently disclosed polymer and that has an optical function. The term "optical function" refers to simple transmission, reflection, refraction, birefringence, or the like.
In terms of the form of the presently disclosed optical film, the optical film may be formed on an alignment substrate that optionally includes an alignment film (i.e., an "alignment substrate/(alignment film)/optical film" form), the optical film may be transferred onto a transparent substrate film or the like differing from an alignment substrate (i.e., a "transparent substrate film/optical film" form), or the optical film may be used as a single layer in a case in which the optical film is self-supporting (i.e., an "optical film" form).
The alignment film and the alignment substrate may the same as a substrate and an alignment film of the subsequently described optically anisotropic body.
The presently disclosed optical film can be produced by methods such as (A) applying a solution of the polymerizable compound-containing mixture or a solution of the polymerizable liquid crystal composition onto an alignment substrate, drying the resultant applied film, performing heat treatment (liquid crystal alignment), and then carrying out photoirradiation and/or heating (polymerization) and (B) applying a solution of a liquid crystal polymer obtained through polymerization of the polymerizable compound-containing mixture or the polymerizable liquid crystal composition onto

an alignment substrate, and optionally drying the resultant applied film.

The presently disclosed optical film can be used for an optically anisotropic body, an alignment film for a liquid crystal display element, a color filter, a low-pass filter, a light polarizing prism, various light filters, and so forth.

The following values $\alpha$ and $\beta$ for the presently disclosed optical film that are determined from retardation at wavelengths of 449.9 nm, 548.5 nm, and 650.2 nm measured by an ellipsometer are preferably within certain ranges. Specifically, the $\alpha$ value is preferably 0.70 to 0.99, and more preferably 0.75 to 0.90, whereas the $\beta$ value is preferably 1.00 to 1.25, and more preferably 1.01 to 1.20.

$$\alpha = (\text{Retardation at } 449.9 \text{ nm})/(\text{Retardation at } 548.5 \text{ nm})$$

$$\beta = (\text{Retardation at } 650.2 \text{ nm})/(\text{Retardation at } 548.5 \text{ nm})$$

(6) Optically anisotropic body

The presently disclosed optically anisotropic body includes a layer having the presently disclosed polymer as a constituent material.

The presently disclosed optically anisotropic body can be obtained by, for example, forming an alignment film on a substrate and then forming a layer formed from the presently disclosed polymer (liquid crystal layer) on the alignment film. Note that the presently disclosed optically anisotropic body may be a body obtained by forming a layer formed from the presently disclosed polymer (liquid crystal layer) directly on a substrate or may be a body composed only of a layer formed from the presently disclosed polymer (liquid crystal layer).

The layer formed from the polymer may be a layer formed from a film-like polymer or may be an aggregate of a powder-like polymer.

The alignment film is formed on the surface of the substrate in order to regulate in-plane alignment of polymerizable liquid crystal compound in one direction.

The alignment film can be obtained by applying a solution containing a polymer such as a polyimide, polyvinyl alcohol, polyester, polyarylate, polyamide imide, or polyetherimide (composition for alignment film) onto a substrate as a film, drying the film, and then performing rubbing or the like in one direction.

The thickness of the alignment film is preferably 0.001 $\mu$m to 5 $\mu$m, and more preferably 0.001 $\mu$m to 1 $\mu$m.

The method by which rubbing is performed is not specifically limited and may, for example, be a method in which the alignment film is rubbed in a given direction using a roll around which cloth or felt formed from synthetic fiber (for example, nylon) or natural fiber (for example, cotton) is wound. It is preferable to wash the alignment film with isopropyl alcohol or the like after the rubbing to remove fine powder (foreign matter) formed during the rubbing and to clean the surface of the alignment film.

Besides rubbing methods, a function of regulating in-plane alignment in one direction can be imparted through a method in which the surface of an alignment film is irradiated with polarized ultraviolet rays.

The substrate on which the alignment film is formed may, for example, be a glass substrate, a substrate formed from a synthetic resin film, or the like. Examples of synthetic resins that may be used include thermoplastic resins such as acrylic resin, polycarbonate resin, polyethersulfone resin, polyethylene terephthalate resin, polyimide resin, polymethyl methacrylate resin, polysulfone resin, polyarylate resin, polyethylene resin, polystyrene resin, polyvinyl chloride resin, cellulose diacetate, cellulose triacetate, and alicyclic olefin polymers.

Examples of alicyclic olefin polymers include cycloolefin random multicomponent copolymers described in JP H05-310845 A and the Specification of US 5179171 A, hydrogenated polymers described in JP H05-97978 A and the Specification of US 5202388 A, and thermoplastic dicyclopentadiene ring-opened polymers and hydrogenated products thereof described in JP H11-124429 A (WO 99/20676 A1).

The method by which a liquid crystal layer formed from the presently disclosed polymer is formed on the alignment film may, for example, be the same as any of the methods described in the section pertaining the presently disclosed polymer (methods ($\alpha$) and ($\beta$)).

Although no specific limitations are placed on the thickness of the liquid crystal layer that is obtained, the thickness is normally 1 $\mu$m to 10 $\mu$m.

The presently disclosed optically anisotropic body may be a retardation plate, a viewing angle enhancement plate, or the like, but is not specifically limited to these types of optically anisotropic bodies.

The following values $\alpha$ and $\beta$ for the presently disclosed optically anisotropic body that are determined from retardation at wavelengths of 449.9 nm, 548.5 nm, and 650.2 nm measured by an ellipsometer are preferably within certain ranges. Specifically, the $\alpha$ value is preferably 0.70 to 0.99, and more preferably 0.75 to 0.90, whereas the $\beta$ value

is preferably 1.00 to 1.25, and more preferably 1.01 to 1.20.

$$\alpha = (\text{Retardation at } 449.9 \text{ nm})/(\text{Retardation at } 548.5 \text{ nm})$$

$$\beta = (\text{Retardation at } 650.2 \text{ nm})/(\text{Retardation at } 548.5 \text{ nm})$$

(7) Polarizer, etc.

The presently disclosed polarizer includes the presently disclosed optically anisotropic body and a polarizing film.

A specific example of the presently disclosed polarizer is a polarizer in which the presently disclosed optically anisotropic body in stacked on a polarizing film either directly or with another layer (for example, a glass sheet) in-between.

No specific limitations are placed on the method by which the polarizing film is produced. Examples of methods by which a PVA polarizing film can be produced include a method in which adsorption of iodine ions by a PVA film is carried out and then uniaxial stretching is performed, a method in which uniaxial stretching of a PVA film is performed and then adsorption of iodine ions is carried out, a method in which adsorption of iodine ions to a PVA film and uniaxial stretching are performed simultaneously, a method in which a PVA film is dyed using a dichroic dye and is then uniaxially stretched, a method in which a PVA film is uniaxially stretched and is then dyed using a dichroic dye, and a method in which dyeing of a PVA film using a dichroic dye and uniaxial stretching are performed simultaneously. Examples of methods by which a polyene polarizing film can be produced include commonly known methods such as a method in which a PVA film is uniaxially stretched and is then heated and dehydrated in the presence of a dehydration catalyst and a method in which a polyvinyl chloride film is uniaxially stretched and is then heated and dehydrated in the presence of a dehydrochlorination catalyst.

In the presently disclosed polarizer, the polarizing film and the presently disclosed optically anisotropic body may be in contact via an adhesive layer formed from an adhesive (inclusive of pressure-sensitive adhesives). The average thickness of the adhesive layer is normally 0.01 μm to 30 μm, and preferably 0.1 μm to 15 μm. The adhesive layer is preferably a layer having a tensile fracture strength of 40 MPa or less according to JIS K7113.

Examples of adhesives that may form the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene-butadiene-styrene copolymer (SBS copolymer) adhesives and adhesives that are hydrogenated products thereof (SEBS copolymers), ethylene adhesives such as ethylene-vinyl acetate copolymers and ethylene-styrene copolymers, and acrylic acid ester adhesives such as ethylene-methyl methacrylate copolymers, ethylene-methyl acrylate copolymers, ethylene-ethyl methacrylate copolymers, and ethylene-ethyl acrylate copolymers.

As a result of the presently disclosed optically anisotropic body being used in the presently disclosed polarizer, the presently disclosed polarizer can be produced at low cost and also has excellent performance in terms of having low reflection luminance and enabling uniform polarized light conversion over a wide wavelength region.

The presently disclosed polarizer can suitably be used to produce a display including a panel and the polarizer or to produce an antireflection film. The display may, for example, be a flat panel display in which a liquid crystal panel is used as the panel or an organic electroluminescence display in which an organic electroluminescence panel is used as the panel.

EXAMPLES

The following provides a more detailed description of the present disclosure through examples. However, the present disclosure is not in any way limited by the following examples.

(Synthesis Example 1) Synthesis of compound 1

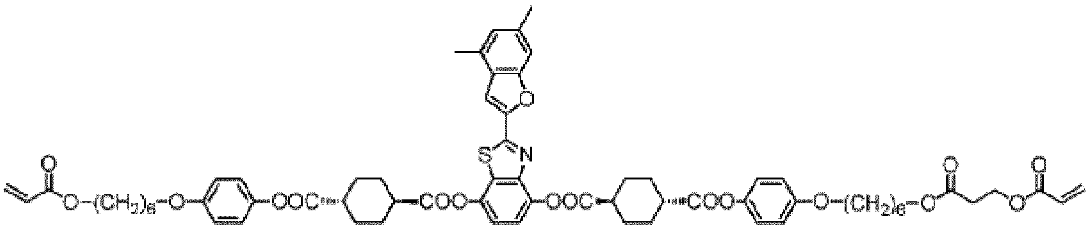

Compound 1

Step 1: Synthesis of intermediate 1

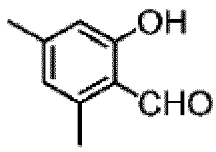

Intermediate 1

A four-necked reaction vessel equipped with a thermometer was charged with 20.0 g (164 mmol) of 3,5-dimethylphenol and 500 mL of acetonitrile in a stream of nitrogen. These materials were dissolved to obtain a solution and then 23.4 g (246 mmol) of magnesium chloride and 58.1 g (574 mmol) of triethylamine were added thereto. The solution was stirred for 30 minutes at 25°C, 14.8 g (492 mmol) of paraformaldehyde was subsequently added, and then the solution was stirred for 3 hours at 75°C. Once the reaction ended, the reaction liquid was cooled to 30°C, 600 mL of 1 M hydrochloric acid was added thereto, and an extraction was performed with 800 mL of diethyl ether. The diethyl ether layer was washed with 300 mL of saturated sodium hydrogen carbonate aqueous solution and 300 mL of saturated saline water, and was then dried with anhydrous magnesium sulfate. Magnesium sulfate was filtered off and diethyl ether was subsequently evaporated under reduced pressure in a rotary evaporator to yield a white solid. The white solid was purified by silica gel column chromatography (hexane:ethyl acetate = 90:10 (volume ratio)) to yield 17.7 g (yield: 71.9 mol%) of intermediate 1 in the form of a white solid.

The structure was identified by $^1$H-NMR.

The $^1$H-NMR spectrum data are shown below.

$^1$H-NMR (500 MHz, $CDCl_3$, TMS, $\delta$ ppm): 11.95 (s, 1H), 10.22 (s, 1H), 6.61 (s, 1H), 6.53 (s, 1H), 2.54 (s, 3H), 2.30 (s, 3H)

Step 2: Synthesis of intermediate 2

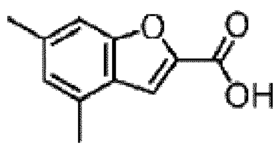

Intermediate 2

A four-necked reaction vessel equipped with a thermometer was charged with 12.0 g (79.9 mmol) of the intermediate 1 synthesized in step 1 and 105 mL of dimethylacetamide in a stream of nitrogen. These materials were dissolved to obtain a solution and then 11.0 g (79.9 mmol) of potassium carbonate was added thereto. The solution was heated to 80°C and then 13.3 g (79.9 mmol) of ethyl bromoacetate was added thereto over 30 minutes. The solution was stirred for 1 hour at 80°C and was then heated to 130°C and further stirred for 1 hour. Thereafter, the reaction liquid was cooled to 30°C, 300 mL of 1 M hydrochloric acid was added thereto, and an extraction was performed with 120 mL of methyl isobutyl ketone. The methyl isobutyl ketone layer was dried with sodium sulfate. Sodium sulfate was then

filtered off and methyl isobutyl ketone was subsequently evaporated under reduced pressure in a rotary evaporator to yield a pale yellow solid. The pale yellow solid was dissolved in 500 mL of ethanol. In addition, 12.0 g (214 mmol) of potassium hydroxide was added to the resultant solution and was stirred therewith for 1 hour at 80°C. Once the reaction ended, ethanol was evaporated under reduced pressure in a rotary evaporator to yield a pale yellow solid. The pale yellow solid was dissolved in 300 mL of water to obtain a solution that was subsequently washed with 300 mL of toluene and 300 mL of heptane. The pH of the solution was adjusted to 3 through addition of 2 M sulfuric acid aqueous solution. Precipitated solid was subsequently filtered off and vacuum dried to yield 12.3 g (yield: 80.9 mol%) of intermediate 2 in the form of a white solid.

The structure was identified by $^1$H-NMR.

The $^1$H-NMR spectrum data are shown below.

$^1$H-NMR (500 MHz, $CDCl_3$, TMS, $\delta$ ppm): 13.42 (brs, 1H), 7.69 (d, 1H, J = 1.0 Hz), 7.30 (s, 1H), 6.98 (s, 1H), 2.48 (s, 3H), 2.41 (s, 3H)

Step 3: Synthesis of intermediate 3

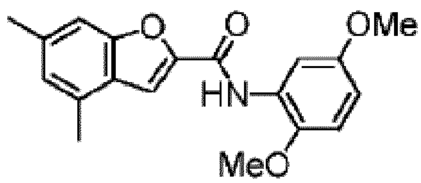

Intermediate 3

A four-necked reaction vessel equipped with a thermometer was charged with 12.0 g (63.1 mmol) of the intermediate 2 synthesized in step 2, 14.5 g (94.6 mmol) of 2,5-dimethoxyaniline, and 120 g of chloroform in a stream of nitrogen. These materials were dissolved to obtain a solution and then a mixed liquid of 13.3 g (69.4 mmol) of 1-(3-dimethylaminopropyl)-3-ethylcarbodiimide hydrochloride and 120 g of chloroform was added and stirred therewith for 3 hours at 25°C. Once the reaction ended, chloroform was evaporated under reduced pressure in a rotary evaporator to yield a pale yellow oily substance. A mixed solution of 200 mL of 1 M hydrochloric acid, 200 mL of water, and 100 mL of methanol was added to the pale yellow oily substance and was stirred therewith at 25°C. Precipitated white solid was filtered off and vacuum dried to yield 16.7 g (yield: 81.2 mol%) of intermediate 3 in the form of a white solid.

The structure was identified by $^1$H-NMR.

The $^1$H-NMR spectrum data are shown below.

$^1$H-NMR (500 MHz, $CDCl_3$, TMS, $\delta$ ppm): 8.28 (d, 1H, J = 3.0 Hz), 7.56 (d, 1H, J = 1.0 Hz), 7.26 (s, 1H), 7.22 (s, 1H), 6.94 (s, 1H), 6.86 (d, 1H, J = 9.0 Hz), 6.64 (dd, 1H, J = 3.0 Hz, 9.0 Hz), 3.97 (s, 3H), 3.81 (s, 3H), 2.51 (s, 3H), 2.49 (s, 3H)

Step 4: Synthesis of intermediate 4

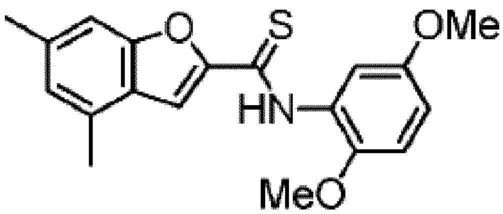

Intermediate 4

A four-necked reaction vessel equipped with a thermometer was charged with 16.0 g (49.2 mmol) of the intermediate 3 synthesized in step 3 and 200 mL of toluene in a stream of nitrogen. These materials were dissolved to obtain a solution and then 12.1 g (23.0 mmol) of 2,4-bis(4-methoxyphenyl)-1,3-dithia-2,4-diphosphetane was added thereto. The solution was then heated under reflux for 4 hours. Once the reaction ended, the reaction liquid was cooled

to 30°C, 400 mL of 1 M sodium hydroxide aqueous solution was added thereto, and an extraction was performed with 500 mL of toluene. A rotary evaporator was used to evaporate 500 mL of toluene from the obtained toluene layer under reduced pressure, and then 500 mL of heptane was added. Precipitated yellow solid was filtered off and vacuum dried to yield 14.7 g (yield: 87.5 mol%) of intermediate 4 in the form of a yellow solid.

The structure was identified by $^1$H-NMR.

The $^1$H-NMR spectrum data are shown below.

$^1$H-NMR (500 MHz, $CDCl_3$, TMS, $\delta$ ppm): 10.45 (s, 1H), 9.13 (d, 1H, J = 3.0 Hz), 7.82 (d, 1H, J = 1.0 Hz), 7.18 (s, 1H), 6.93 (s, 1H), 6.91 (d, 1H, J = 9.0 Hz), 6.77 (dd, 1H, J = 3.0 Hz, 9.0 Hz), 3.97 (s, 3H), 3.83 (s, 3H), 2.51 (s, 3H), 2.46 (s, 3H)

Step 5: Synthesis of intermediate 5

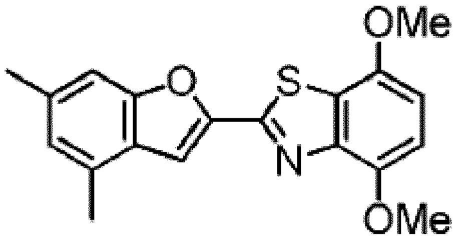

Intermediate 5

A four-necked reaction vessel equipped with a thermometer was charged with 13.2 g (38.6 mmol) of the intermediate 4 synthesized in step 4, 220 g of water, and 11.9 g (212 mmol) of potassium hydroxide in a stream of nitrogen and was stirred under ice cooling. Next, 29.2 g (88.8 mmol) of potassium ferricyanide and 12 g of methanol were added to the resultant mixed liquid, and were heated to 60°C and stirred for 6 hours therewith. Once the reaction ended, the reaction liquid was cooled to 30°C. Precipitated yellow solid was filtered off and vacuum dried to yield 10.2 g (yield: 76.8 mol%) of intermediate 5 in the form of a yellow solid.

The structure was identified by $^1$H-NMR.

The $^1$H-NMR spectrum data are shown below.

$^1$H-NMR (500 MHz, $CDCl_3$, TMS, $\delta$ ppm): 7.65 (d, 1H, J = 1.0 Hz), 7.21 (s, 1H), 6.91 (s, 1H), 6.84 (d, 1H, J = 8.5 Hz), 6.76 (d, 1H, J = 8.5 Hz), 4.04 (s, 3H), 3.97 (s, 3H), 2.51 (s, 3H), 2.46 (s, 3H)

Step 6: Synthesis of intermediate 6

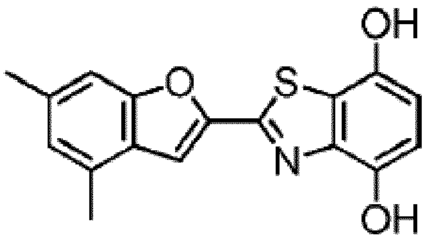

Intermediate 6

A four-necked reaction vessel equipped with a thermometer was charged with 7.2 g (21.2 mmol) of the intermediate 5 synthesized in step 5 and 72 g of pyridine hydrochloride in a stream of nitrogen and was stirred for 4 hours at 180°C. Once the reaction ended, the reaction liquid was cooled to 30°C and 300 g of water was added thereto. Precipitated solid was filtered off and washed with 30 g of water, 30 g of toluene, and 30 g of hexane. The resultant solid was vacuum dried to yield 6.38 g (yield: 96.6 mol%) of intermediate 6 in the form of a yellow solid.

The structure was identified by $^1$H-NMR.

The $^1$H-NMR spectrum data are shown below.

$^1$H-NMR (500 MHz, DMSO-$d_6$, TMS, $\delta$ ppm): 9.91 (s, 1H), 9.59 (brs, 1H), 7.76 (d, 1H, J = 1.0 Hz), 7.36 (s, 1H), 6.99 (s, 1H), 6.79 (d, 1H, J = 8.5 Hz), 6.74 (d, 1H, J = 8.5 Hz), 2.53 (s, 3H), 2.43 (s, 3H)

Step 7: Synthesis of intermediate 7

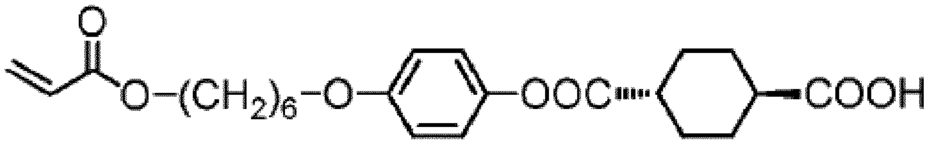

Intermediate 7

A three-necked reaction vessel equipped with a thermometer was charged with 17.98 g (104.42 mmol) of trans-1,4-cyclohexanedicarboxylic acid and 180 mL of tetrahydrofuran (THF) in a stream of nitrogen. In addition, 6.58 g (57.43 mmol) of methanesulfonyl chloride was added into the reaction vessel and the reaction vessel was immersed in a water bath to attain a reaction liquid internal temperature of 20°C. Next, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C.

Next, 0.64 g (5.22 mmol) of 4-(dimethylamino)pyridine and 13.80 g (52.21 mmol) of 4-(6-acryloyloxy-hex-1-yloxy)phenol (produced by DKSH) were added to the resultant reaction liquid, and the reaction vessel was immersed in a water bath once again to attain a reaction liquid internal temperature of 15°C. Thereafter, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25 °C. Once the reaction ended, 1,000 mL of distilled water and 100 mL of saturated saline water were added to the reaction liquid, and two extractions were performed with 400 mL of ethyl acetate. The organic layers were collected and were dried with anhydrous sodium sulfate, and then sodium sulfate was filtered off. Solvent was evaporated from the filtrate in a rotary evaporator and then the resultant residue was purified by silica gel column chromatography (THF:toluene = 1:9 (volume ratio)) to yield 14.11 g (yield: 65 mol%) of intermediate 7 in the form of a white solid.

The structure was identified by $^1$H-NMR. The results are shown below.

$^1$H-NMR (500 MHz, DMSO-$d_6$, TMS, $\delta$ ppm): 12.12 (s, 1H), 6.99 (d, 2H, J = 9.0 Hz), 6.92 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.94 (t, 2H, J = 6.5 Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H)

Step 8: Synthesis of intermediate 8

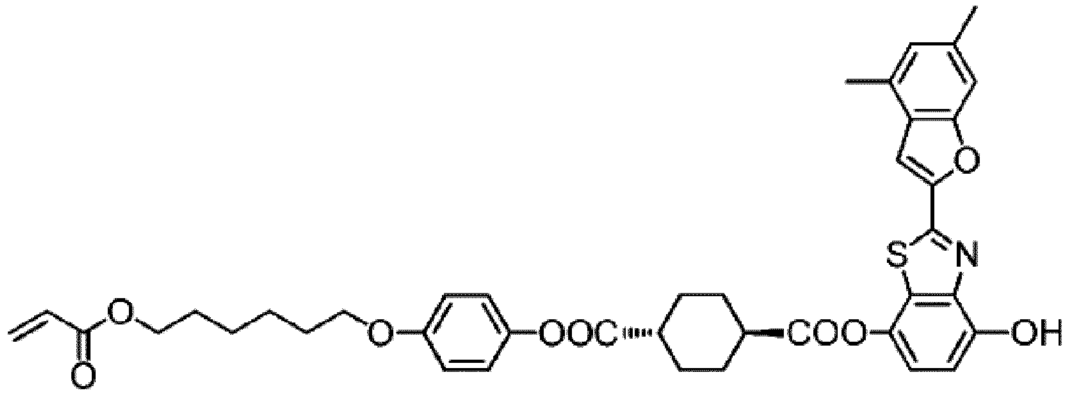

Intermediate 8

A three-necked reaction vessel equipped with a thermometer was charged with 4.0 g (12.8 mmol) of the intermediate 6 synthesized in step 6 and 160 mL of THF in a stream of nitrogen and was cooled to 0°C. Next, 6.44 g (15.4 mmol) of the intermediate 7 synthesized in step 7, 156 mg (1.28 mmol) of 4-dimethylaminopyridine, and 1.94 g (15.4 mmol) of N,N'-diisopropylcarbodiimide were added to the resultant solution and were stirred therewith for 1 hour at room temperature. Once the reaction ended, 200 mL of water was added to the reaction liquid and an extraction was performed with 400 mL of ethyl acetate. The resultant ethyl acetate layer was dried with anhydrous sodium sulfate, and then sodium sulfate was filtered off. Concentrating was performed using a rotary evaporator and purification of the resultant residue was subsequently performed by silica gel column chromatography (toluene:ethyl acetate = 90:10 (volume ratio)) to yield 1.29 g (yield: 14.1 mol%) of intermediate 8 in the form of a flesh-colored solid.

The structure of the target was identified by $^1$H-NMR. The results are shown below.

$^1$H-NMR (500 MHz, $CDCl_3$, TMS, $\delta$ ppm): 7.53 (d, 1H, J = 1.0 Hz), 7.21 (s, 1H), 7.10 (d, 1H, J = 9.0 Hz), 6.98-7.01 (m, 4H), 6.94 (s, 1H), 6.88 (d, 2H, J = 9.0 Hz), 6.41 (dd, 1H, J = 1.5 Hz, 17.5 Hz), 6.13 (dd, 1H, J = 10.5 Hz, 17.5 Hz), 6.13 (dd, 1H, J = 1.5 Hz, 10.5 Hz), 4.18 (t, 2H, J = 7.0 Hz), 3.95 (t, 2H, J = 6.5 Hz), 2.53 (s, 3H), 2.47 (s, 3H), 2.32-2.43 (m, 4H), 1.67-1.82 (m, 10H), 1.45-1.56 (m, 4H)

Step 9: Synthesis of intermediate 9

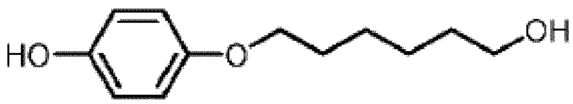

Intermediate 9

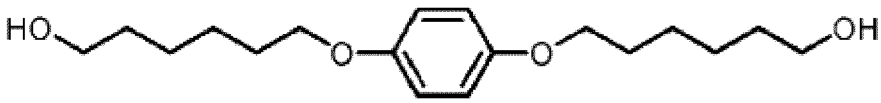

By-product 9

A three-necked reaction vessel equipped with a condenser and a thermometer was charged with 104.77 g (0.9515 mol) of hydroquinone, 100 g (0.7320 mol) of 6-chlorohexanol, 500 g of distilled water, and 100 g of o-xylene in a stream of nitrogen. The entire contents of the reaction vessel were stirred while 35.15 g (0.8784 mol) of sodium hydroxide was added gradually over 20 minutes such that the temperature of the contents did not exceed 40°C. After addition of the sodium hydroxide was completed, the contents were heated and were allowed to react for 12 hours under reflux conditions (96°C).

Once the reaction ended, the temperature of the reaction liquid was lowered to 80°C, 200 g of distilled water was added, and then the reaction liquid was cooled to 10°C to cause precipitation of crystals. Solid-liquid separation

was performed through filtration of the precipitated crystals. The obtained crystals were washed with 500 g of distilled water and vacuum dried to yield 123.3 g of brown crystals.

The content ratio (molar ratio) of compounds contained in the brown crystals was determined to be hydroquinone/intermediate 9/by-product 9 = 1.3/90.1/8.1 through analysis of the brown crystals by high-performance liquid chromatography. This mixture was used in step 10 without purification.

Step 10: Synthesis of intermediate 10

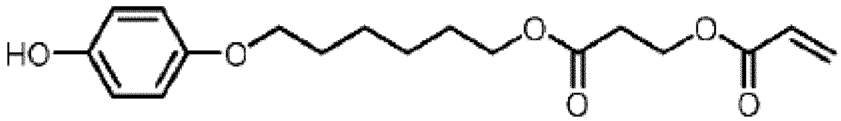

Intermediate 10

A three-necked reaction vessel equipped with a thermometer and a condenser having a Dean-Stark tube was charged with 10.00 g of the brown crystals containing intermediate 9 synthesized in step 9, 100 g of toluene, and 0.105 g (0.476 mmol) of 2,6-di-t-butyl-p-cresol in a stream of nitrogen, and the entire contents of the reaction vessel were stirred. The resultant solution was heated to 80°C, and 20.56 g (0.1427 mol) of 2-carboxyethyl acrylate and 1.37 g (14.3 mmol) of methanesulfonic acid were added thereto. A dehydration reaction was carried out under reflux conditions (110°C) for 2 hours while removing produced water. Next, the reaction liquid was cooled to 30°C and 500 g of distilled water was added thereto. The entire contents of the reaction vessel was stirred and were subsequently left at rest. The organic layer was separated and 500 g of 5% saline water was added thereto. Liquid separation was performed, and the organic layer was collected and then dried with anhydrous sodium sulfate. Sodium sulfate was subsequently filtered off. Concentrating was performed using a rotary evaporator and then purification was performed by silica gel column chromatography (toluene:ethyl acetate = 8:1 (volume ratio)) to yield 7.93 g (yield: 40 mol%) of intermediate 10 in the form of a white solid through the total of steps 9 and 10.

The structure of the target was identified by $^1$H-NMR. The results are shown below.

$^1$H-NMR (500 MHz, $CDCl_3$, TMS, $\delta$ ppm): 6.77 (d, 2H, J = 9.0 Hz), 6.76 (d, 2H, J = 9.0 Hz), 6.41 (dd, 1H, J = 1.5 Hz, 17.5 Hz), 6.11 (dd, 1H, J = 10.5 Hz, 17.5 Hz), 5.83 (dd, 1H, J = 1.5 Hz, 10.5 Hz), 4.83 (s, 1H), 4.44 (t, 2H, J = 6.5 Hz), 4.13 (t, 2H, J = 6.5 Hz), 3.89 (t, 2H, J = 6.5 Hz), 2.69 (t, 2H, J = 6.5 Hz), 1.71-1.80 (m, 2H), 1.62-1.70 (m, 2H), 1.36-1.52 (m, 4H)

Step 11: Synthesis of intermediate 11

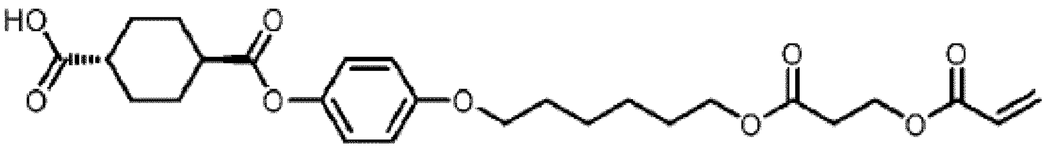

Intermediate 11

A three-necked reaction vessel equipped with a thermometer was charged with 3.58 g (0.0208 mol) of trans-1,4-cyclohexanedicarboxylic acid and 25 mL of THF in a stream of nitrogen. Next, 1.25 g (0.0109 mol) of methanesulfonyl chloride was added into the reaction vessel and the reaction vessel was immersed in a water bath to attain a reaction liquid internal temperature of 5°C. Dropwise addition of 1.15 g (0.0114 mol) of triethylamine was performed over 15 minutes such that the reaction liquid internal temperature was held at 15°C or lower. The reaction liquid was stirred for 1 hour at 5°C, and then 0.127 g (1.04 mmol) of 4-(dimethylamino)pyridine and 3.51 g (0.0104 mol) of intermediate 10 synthesized in step 10 were added thereto. Dropwise addition of 1.15 g (0.0114 mol) of triethylamine was performed over 15 minutes such that the reaction liquid internal temperature was held at 15°C or lower. Thereafter, the reaction liquid was allowed to react for 2 hours at 25°C. Once the reaction ended, 300 mL of distilled water and 30 mL of saturated

saline water were added to the reaction liquid, and two extractions were performed with 200 mL of chloroform. The resultant organic layers were dried with anhydrous sodium sulfate, and then sodium sulfate was filtered off. Concentrating was performed using a rotary evaporator and then purification was performed by silica gel column chromatography (chloroform:THF = 95:5 (volume ratio)) to yield 2.41 g (yield: 47 mol%) of intermediate 11 in the form of a white solid.

The structure of the target was identified by $^1$H-NMR. The results are shown below.

$^1$H-NMR (500 MHz, $CDCl_3$, TMS, $\delta$ ppm): 6.96 (d, 2H, J = 9.0 Hz), 6.86 (d, 2H, J = 9.0 Hz), 6.41 (dd, 1H, J = 1.5 Hz, 17.5 Hz), 6.11 (dd, 1H, J = 10.5 Hz, 17.5 Hz), 5.83 (dd, 1H, J = 1.5 Hz, 10.5 Hz), 4.44 (t, 2H, J = 6.5 Hz), 4.13 (t, 2H, J = 6.5 Hz), 3.93 (t, 2H, J = 6.5 Hz), 2.69 (t, 2H, J = 6.5 Hz), 2.47-2.57 (m, 1H), 2.34-2.43 (m, 1H), 2.12-2.28 (m, 4H), 1.73-1.82 (m, 2H), 1.36-1.71 (m, 10H)

Step 12: Synthesis of compound 1

A four-necked reaction vessel equipped with a thermometer was charged with 975 mg (1.37 mmol) of the intermediate 8 synthesized in step 8 and 20 mL of chloroform in a stream of nitrogen. Next, 810 mg (1.65 mmol) of the intermediate 11 synthesized in step 11 and 16.5 mg (0.135 mmol) of 4-dimethylaminopyridine were added to the resultant solution. The solution was cooled to 0°C and then 207 mg (1.65 mmol) of N,N'-diisopropylcarbodiimide was added and was stirred therewith for 1.5 hours at room temperature. Once the reaction ended, the reaction liquid was filtered using a filter medium precoated with silica gel and was subsequently concentrated under reduced pressure to obtain a residue to which 80 mL of methanol was then added. Precipitated white solid was filtered off and vacuum dried to yield 1.35 g (yield: 82.8 mol%) of compound 1 in the form of a white solid.

The structure was identified by $^1$H-NMR. The results are shown below.

$^1$H-NMR (400 MHz, $CDCl_3$, TMS, $\delta$ ppm): 7.53 (s, 1H), 7.22 (s, 2H), 7.20 (s, 1H), 6.99 (d, 4H, J = 9.0 Hz), 6.94 (s, 1H), 6.88 (d, 4H, J = 9.0 Hz), 6.37-6.43 (m, 2H), 6.12 (dd, 1H, J = 11.0 Hz, 17.6 Hz), 6.11 (dd, 1H, J = 10.8 Hz, 17.6 Hz), 5.83 (dd, 1H, J = 1.6 Hz, 11.0 Hz), 5.81 (dd, 1H, J = 1.6 Hz, 10.8 Hz), 4.43 (t, 2H, J = 6.4 Hz), 4.17 (t, 2H, J = 6.8 Hz), 4.13 (t, 2H, J = 6.4 Hz), 3.94 (t, 4H, J = 6.4 Hz), 2.84 (tt, 1H, J = 4.0 Hz, 11.6 Hz), 2.59-2.75 (m, 5H), 2.54 (s, 3H), 2.41-2.48 (m, 5H), 2.29-2.39 (m, 6H), 1.64-1.87 (m, 16H), 1.38-1.55 (m, 8H)

(Synthesis Example 2) Synthesis of compound 2

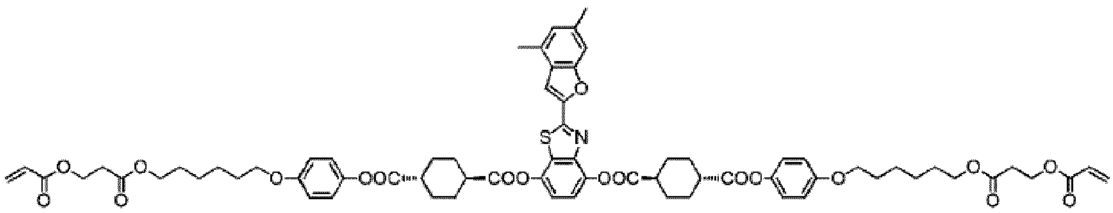

Compound 2

A four-necked reaction vessel equipped with a thermometer was charged with 0.5 g (1.61 mmol) of the intermediate 6 synthesized in step 6 of Synthesis Example 1 and 30 mL of chloroform in a stream of nitrogen. Next, 1.65 g (3.37 mmol) of the intermediate 11 synthesized in step 11 of Synthesis Example 1 and 39.3 mg (0.32 mmol) of 4-dimethylaminopyridine were added to the resultant solution. The solution was cooled to 0°C, and then 486 mg (3.85 mmol) of N,N'-diisopropylcarbodiimide was added and was stirred therewith for 1 hour at room temperature. Once the reaction ended, the reaction liquid was filtered using a filter medium precoated with silica gel and was subsequently concentrated under reduced pressure to obtain a residue to which 50 mL of methanol was then added. Precipitated white solid was filtered off and vacuum dried to yield 1.20 g (yield: 59.3 mol%) of compound 2 in the form of a white solid.

The structure was identified by $^1$H-NMR. The results are shown below.

$^1$H-NMR (500 MHz, $CDCl_3$, TMS, $\delta$ ppm): 7.54 (d, 1H, J = 1.0 Hz), 7.23 (s, 2H), 7.21 (s, 1H), 7.001 (d, 2H, J = 9.0 Hz), 6.996 (d, 2H, J = 9.0 Hz), 6.94 (s, 1H), 6.89 (d, 4H, J = 9.0 Hz), 6.41 (dd, 2H, J = 1.5 Hz, 17.5 Hz), 6.11 (dd, 2H, J = 10.5 Hz, 17.5 Hz), 5.83 (dd, 2H, J = 1.5 Hz, 10.5 Hz), 4.44 (t, 4H, J = 6.5 Hz), 4.14 (t, 4H, J = 6.5 Hz), 3.95 (t, 4H, J = 6.5 Hz), 2.85 (tt, 1H, J = 3.5 Hz, 11.5 Hz), 2.56-2.77 (m, 7H), 2.55 (s, 3H), 2.42-2.51 (m, 5H), 2.29-2.40 (m, 6H), 1.65-1.87 (m, 16H), 1.39-1.57 (m, 8H)

(Synthesis Example 3) Synthesis of compound 3

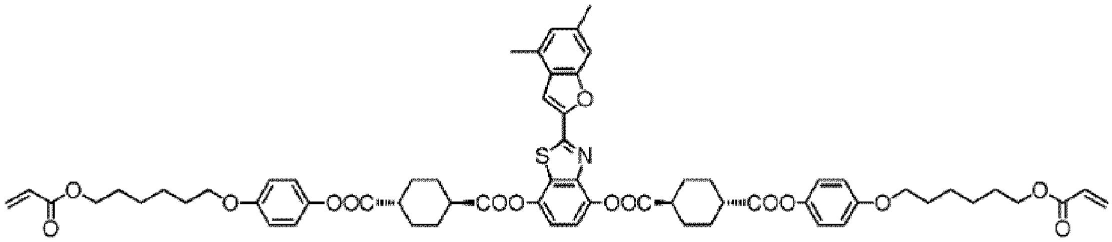

Compound 3

A four-necked reaction vessel equipped with a thermometer was charged with 1.00 g (3.21 mmol) of the intermediate 6 synthesized in step 6 of Synthesis Example 1 and 50 mL of chloroform in a stream of nitrogen. Next, 2.96 g (7.07 mmol) of the intermediate 7 synthesized in step 7 of Synthesis Example 1 and 39.2 mg (0.321 mmol) of 4-dimethylaminopyridine were added to the resultant solution. The solution was cooled to 0°C and then 972 mg (7.70 mmol) of N,N'-diisopropylcarbodiimide was added and was stirred therewith for 1.5 hours at room temperature. Once the reaction ended, the reaction liquid was filtered using a filter medium precoated with silica gel, was subsequently concentrated under reduced pressure, and then the resultant residue was purified by silica gel column chromatography (chloroform:ethyl acetate = 90:10 (volume ratio)) to yield 2.84 g (yield: 79.5%) of compound 3 in the form of a white solid.

The structure was identified by $^1H$-NMR.

The $^1H$-NMR spectrum data are shown below.

$^1H$-NMR (500 MHz, $CDCl_3$, TMS, $\delta$ ppm): 7.53 (d, 1H, J = 1.0 Hz), 7.23 (s, 2H), 7.21 (s, 1H), 6.999 (d, 2H, J = 9.0 Hz), 6.995 (d, 2H, J = 9.0 Hz), 6.94 (s, 1H), 6.89 (d, 4H, J = 9.0 Hz), 6.40 (dd, 2H, J = 1.5 Hz, 17.5 Hz), 6.12 (dd, 2H, J = 10.5 Hz, 17.5 Hz), 5.82 (dd, 2H, J = 1.5 Hz, 10.5 Hz), 4.18 (t, 4H, J = 7.0 Hz), 3.95 (t, 4H, J = 6.5 Hz), 2.84 (tt, 1H, J = 3.5 Hz, 12.0 Hz), 2.59-2.75 (m, 3H), 2.54 (s, 3H), 2.47 (s, 3H), 2.42-2.46 (m, 2H), 2.31-2.41 (m, 6H), 1.69-1.87 (m, 16H), 1.41-1.57 (m, 8H)

(Synthesis Example 4) Synthesis of compound 4

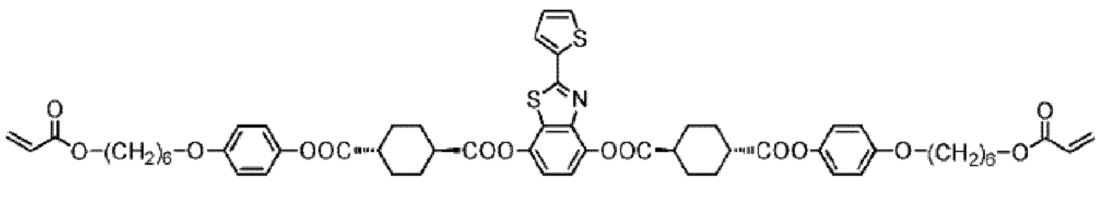

Compound 4

Step 1: Synthesis of intermediate 12

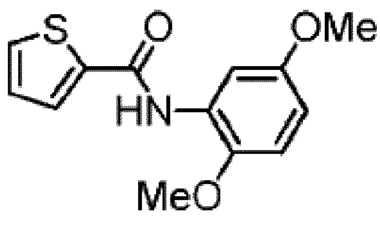

Intermediate 12

A four-necked reaction vessel equipped with a thermometer was charged with 5.00 g (34.1 mmol) of 2-thenoyl chloride, 5.22 g (34.1 mmol) of 2,5-dimethoxyaniline, and 50 g of chloroform in a stream of nitrogen. These materials were dissolved to obtain a solution and then 6.90 g (68.2 mmol) of triethylamine was added and stirred therewith for 2 hours at 60°C. Once the reaction ended, 50 g of water was added and then an extraction was performed with 100 mL of chloroform. A rotary evaporator was used to evaporate 100 mL of chloroform from the obtained chloroform layer under reduced pressure, and then 100 mL of heptane was added. Precipitated pale yellow solid was filtered off and vacuum dried to yield 7.79 g (yield: 86.7%) of intermediate 12 in the form of a pale yellow solid.

The structure was identified by $^1$H-NMR.

The $^1$H-NMR spectrum data are shown below.

$^1$H-NMR (500 MHz, $CDCl_3$, TMS, $\delta$ ppm): 8.45 (s, 1H), 8.20 (d, 1H, J = 3.0 Hz), 7.61 (dd, 1H, J = 1.0 Hz, 3.5 Hz), 7.54 (dd, 1H, J = 1.0 Hz, 5.0 Hz), 7.13 (dd, 1H, J = 3.5 Hz, 5.0 Hz), 6.83 (d, 1H, J = 9.0 Hz), 6.61 (dd, 1H, J = 3.0 Hz, 9.0 Hz), 3.89 (s, 3H), 3.81 (s, 3H)

Step 2: Synthesis of intermediate 13

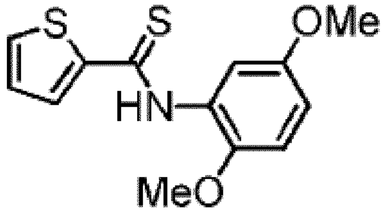

Intermediate 13

A four-necked reaction vessel equipped with a thermometer was charged with 7.00 g (26.6 mmol) of the intermediate 12 synthesized in step 1 and 100 mL of toluene in a stream of nitrogen. These materials were dissolved to obtain a solution and then 6.45 g (15.9 mmol) of 2,4-bis(4-methoxyphenyl)-1,3-dithia-2,4-diphosphetane was added thereto. The solution was then heated under reflux for 4 hours. Once the reaction ended, the reaction liquid was cooled to 30°C, 400 mL of 1 M sodium hydroxide aqueous solution was added thereto, and an extraction was performed with 500 mL of toluene. Toluene was evaporated from the resultant toluene layer under reduced pressure in a rotary evaporator to yield an oily substance. The obtained oily substance was purified by silica gel column chromatography (toluene:ethyl acetate = 90:10 (volume ratio)) to yield 7.07 g (yield: 95.2%) of intermediate 13 in the form of an orange oily substance.

The structure was identified by $^1$H-NMR.

The $^1$H-NMR spectrum data are shown below.

$^1$H-NMR (500 MHz, $CDCl_3$, TMS, $\delta$ ppm): 9.71 (s, 1H), 8.88 (s, 1H), 7.52 (dd, 1H, J = 1.0 Hz, 5.0 Hz), 7.47 (dd, 1H, J = 1.0 Hz, 4.0 Hz), 7.09 (dd, 1H, J = 4.0 Hz, 5.0 Hz), 6.86 (d, 1H, J = 9.0 Hz), 6.70 (dd, 1H, J = 3.0 Hz, 9.0 Hz), 3.89 (s, 3H), 3.78 (s, 3H)

Step 3: Synthesis of intermediate 14

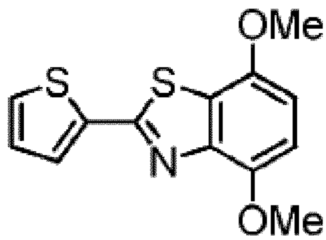

Intermediate 14

A four-necked reaction vessel equipped with a thermometer was charged with 7.00 g (25.1 mmol) of the intermediate 13 synthesized in step 2, 120 g of water, and 8.20 g (146 mmol) of potassium hydroxide in a stream of

nitrogen and was stirred under ice cooling. Next, 21.9 g (66.5 mmol) of potassium ferricyanide and 6 g of methanol were added to the resultant mixed liquid, and were heated to 25°C and stirred for 15 hours therewith. Once the reaction ended, precipitated yellow solid was filtered off and vacuum dried to yield 3.40 g (yield: 46.1%) of intermediate 14 in the form of a yellow solid.

The structure was identified by $^1$H-NMR.

The $^1$H-NMR spectrum data are shown below.

$^1$H-NMR (400 MHz, $CDCl_3$, TMS, $\delta$ ppm): 7.67 (dd, 1H, J = 1.2 Hz, 3.6 Hz), 7.46 (dd, 1H, J = 1.2 Hz, 5.2 Hz), 7.11 (dd, 1H, J = 3.6 Hz, 5.2 Hz), 6.82 (d, 1H, J = 8.8 Hz), 7.30 (d, 1H, J = 8.8 Hz), 4.02 (s, 3H), 3.95 (s, 3H)

Step 4: Synthesis of intermediate 15

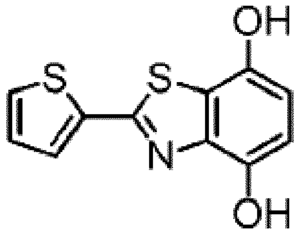

Intermediate 15

A four-necked reaction vessel equipped with a thermometer was charged with 2.10 g (7.72 mmol) of the intermediate 14 synthesized in step 3 and 50 mL of toluene in a stream of nitrogen. These materials were dissolved and then cooled to 0°C. Next, 46.3 mL (46.3 mmol) of 1 M boron tribromide dichloromethane solution was added to the resultant solution and was stirred therewith for 1 hour. Once the reaction ended, the reaction liquid was added into 200 mL of water. Precipitated solid was filtered off and vacuum dried to yield 1.79 g (yield: 93.2%) of intermediate 15 in the form of a yellow solid.

The structure was identified by $^1$H-NMR.

The $^1$H-NMR spectrum data are shown below.

$^1$H-NMR (500 MHz, DMSO-$d_6$, TMS, $\delta$ ppm): 9.82 (s, 1H), 9.48 (s, 1H), 7.83 (dd, 1H, J = 1.0 Hz, 5.0 Hz), 7.78 (dd, 1H, J = 1.0 Hz, 3.5 Hz), 7.23 (dd, 1H, J = 3.5 Hz, 5.0 Hz), 6.74 (d, 1H, J = 8.5 Hz), 6.68 (d, 1H, J = 8.5 Hz)

Step 5: Synthesis of compound 4

A four-necked reaction vessel equipped with a thermometer was charged with 1.50 g (6.02 mmol) of the intermediate 15 synthesized in step 4 and 90 mL of chloroform in a stream of nitrogen. Next, 5.29 g (12.63 mmol) of the intermediate 7 synthesized in step 7 of Synthesis Example 1 and 147 mg (1.20 mmol) of 4-dimethylaminopyridine were added to the resultant solution. The solution was cooled to 0°C and then 1.82 g (14.5 mmol) of N,N'-diisopropylcarbodiimide was added and stirred therewith for 1.5 hours at room temperature. Once the reaction ended, the reaction liquid was filtered using a filter medium precoated with silica gel and was subsequently concentrated under reduced pressure to obtain a residue to which 150 mL of methanol was then added. Precipitated solid was filtered off and vacuum dried to yield 4.69 g (yield: 74.2%) of compound 4 in the form of a white solid.

The structure was identified by $^1$H-NMR.

The $^1$H-NMR spectrum data are shown below.

$^1$H-NMR (500 MHz, $CDCl_3$, TMS, $\delta$ ppm): 7.63 (dd, 1H, J = 1.0 Hz, 3.5 Hz), 7.51 (dd, 1H, J = 1.0 Hz, 5.0 Hz), 7.18 (s, 2H), 7.12 (dd, 1H, J = 3.5 Hz, 5.0 Hz), 6.993 (d, 2H, J = 9.0 Hz), 6.987 (d, 2H, J = 9.0 Hz), 6.88 (d, 4H, J = 9.0 Hz), 6.40 (dd, 2H, J = 1.5 Hz, 17.5 Hz), 6.12 (dd, 2H, J = 10.0 Hz, 17.5 Hz), 5.82 (dd, 2H, J = 1.5 Hz, 10.0 Hz), 4.17 (t, 4H, J = 6.5 Hz), 3.94 (t, 4H, J = 6.5 Hz), 2.79 (tt, 1H, J = 3.5 Hz, 11.5 Hz), 2.58-2.71 (m, 3H), 2.42-2.45 (m, 2H), 2.31-2.36 (m, 6H), 1.66-1.89 (m, 16H), 1.42-1.54 (m, 8H)

(Synthesis Example 5) Synthesis of compound 5

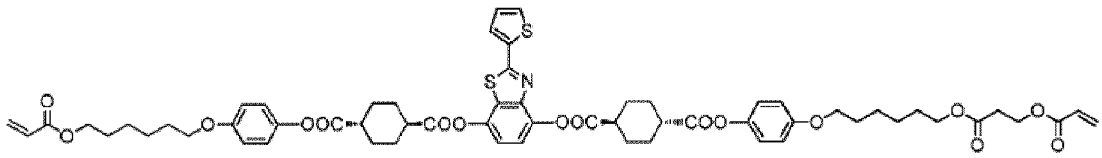

Compound 5

Step 1: Synthesis of intermediate 16

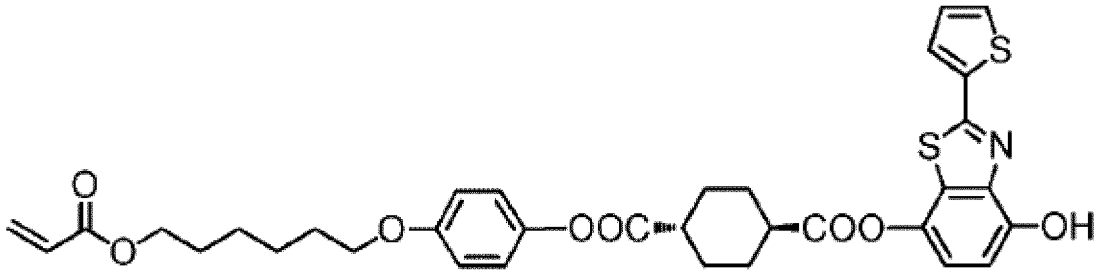

Intermediate 16

A three-necked reaction vessel equipped with a thermometer was charged with 4.0 g (16.04 mmol) of the intermediate 15 synthesized in step 4 of Synthesis Example 4 and 160 mL of THF in a stream of nitrogen, and was then cooled to 0°C. Next, 8.05 g (19.25 mmol) of the intermediate 7 synthesized in step 7 of Synthesis Example 1, 196 mg (1.60 mmol) of 4-dimethylaminopyridine, and 2.43 g (19.25 mmol) of N,N'-diisopropylcarbodiimide were added to the resultant solution and were stirred therewith for 1 hour at room temperature. Once the reaction ended, 200 mL of water was added to the reaction liquid and an extraction was performed with 400 mL of ethyl acetate. The resultant ethyl acetate layer was dried with anhydrous sodium sulfate, and then sodium sulfate was filtered off. Concentrating was performed using a rotary evaporator and then purification of the resultant residue was performed by silica gel column chromatography (toluene:ethyl acetate = 90:10 (volume ratio)) to yield 1.55 g (yield: 14.9 mol%) of intermediate 16 in the form of a pale ocher solid.

Step 2: Synthesis of compound 5

A four-necked reaction vessel equipped with a thermometer was charged with 1.5 g (2.31 mmol) of the intermediate 16 obtained in step 1 and 30 mL of chloroform in a stream of nitrogen. Next, 1.36 g (2.77 mmol) of the intermediate 11 synthesized in step 11 of Synthesis Example 1 and 33.8 mg (0.277 mmol) of 4-dimethylaminopyridine were added to the resultant solution. The solution was cooled to 0°C, and then 350 mg (2.77 mmol) of N,N'-diisopropylcarbodiimide was added and was stirred therewith for 2 hours at room temperature. Once the reaction ended, the reaction liquid was filtered using a filter medium precoated with silica gel and was subsequently concentrated under reduced pressure to obtain a residue to which 100 mL of methanol was then added. Precipitated white solid was filtered off and vacuum dried to yield 1.88 g (yield: 72.5 mol%) of compound 5 in the form of a white solid.

The structure was identified by $^1$H-NMR. The results are shown below.

$^1$H-NMR (500 MHz, $CDCl_3$, TMS, $\delta$ ppm): 7.63 (dd, 1H, J = 1.0 Hz, 3.5 Hz), 7.50 (dd, 1H, J = 1.0 Hz, 5.0 Hz), 7.17 (s, 2H), 7.11 (dd, 1H, J = 3.5 Hz, 5.0 Hz), 6.99 (d, 4H, J = 9.0 Hz), 6.88 (d, 4H, J = 9.0 Hz), 6.40 (dd, 2H, J = 1.5 Hz, 17.5 Hz), 6.12 (dd, 2H, J = 10.0 Hz, 17.5 Hz), 5.82 (dd, 2H, J = 1.5 Hz, 10.0 Hz), 4.43 (t, 2H, J = 6.4 Hz), 4.17 (t, 4H, J = 6.5 Hz), 3.93 (t, 4H, J = 6.5 Hz), 2.79 (tt, 1H, J = 3.5 Hz, 11.5 Hz), 2.58-2.71 (m, 5H), 2.41-2.46 (m, 2H), 2.29-2.34 (m, 6H), 1.62-1.89 (m, 16H), 1.40-1.52 (m, 8H)

(Synthesis Example 6) Synthesis of compound 6

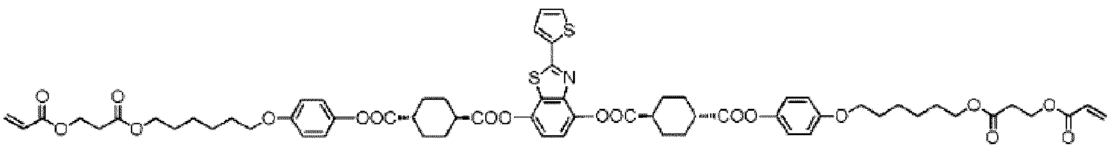

Compound 6

A four-necked reaction vessel equipped with a thermometer was charged with 1.0 g (4.01 mmol) of the intermediate 15 synthesized in step 4 of Synthesis Example 4 and 60 mL of chloroform in a stream of nitrogen. Next, 4.13 g (8.42 mmol) of the intermediate 11 synthesized in step 11 of Synthesis Example 1 and 98 mg (0.80 mmol) of 4-dimethylaminopyridine were added to the resultant solution. The solution was cooled to 0°C and then 1.21 g (9.58 mmol) of N,N'-diisopropylcarbodiimide was added and stirred therewith for 1 hour at room temperature. Once the reaction ended, the reaction liquid was filtered using a filter medium precoated with silica gel and was subsequently concentrated under reduced pressure to obtain a residue to which 100 mL of methanol was then added. Precipitated white solid was filtered off and vacuum dried to yield 3.51 g (yield: 73.3 mol%) of compound 6 in the form of a white solid.

The structure was identified by $^1$H-NMR. The results are shown below.

$^1$H-NMR (500 MHz, $CDCl_3$, TMS, $\delta$ ppm): 7.64 (dd, 1H, J = 1.0 Hz, 3.5 Hz), 7.52 (dd, 1H, J = 1.0 Hz, 5.0 Hz), 7.18 (s, 2H), 7.13 (dd, 1H, J = 3.5 Hz, 5.0 Hz), 6.994 (d, 2H, J = 9.0 Hz), 6.988 (d, 2H, J = 9.0 Hz), 6.88 (d, 4H, J = 9.0 Hz), 6.41 (dd, 2H, J = 1.5 Hz, 17.5 Hz), 6.11 (dd, 2H, J = 10.0 Hz, 17.5 Hz), 5.83 (dd, 2H, J = 1.5 Hz, 10.0 Hz), 4.44 (t, 4H, J = 6.5 Hz), 4.13 (t, 4H, J = 6.5 Hz), 3.94 (t, 4H, J = 6.5 Hz), 2.80 (tt, 1H, J = 3.5 Hz, 11.5 Hz), 2.55-2.73 (m, 7H), 2.42-2.45 (m, 2H), 2.29-2.35 (m, 6H), 1.63-1.89 (m, 16H), 1.41-1.54 (m, 8H)

(Synthesis Example 7) Synthesis of mixture 1

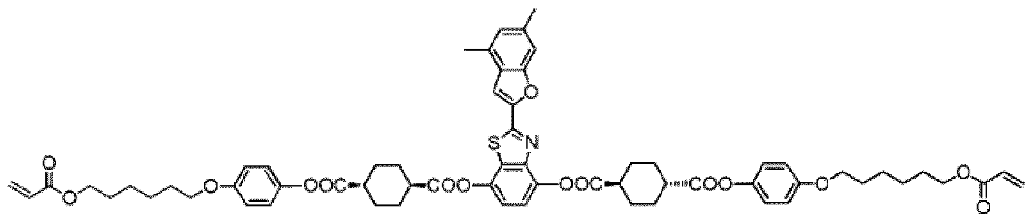

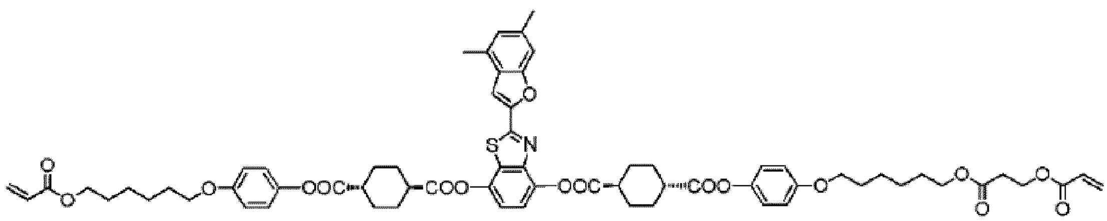

Mixture 1

Step 1: Synthesis of intermediate mixture A

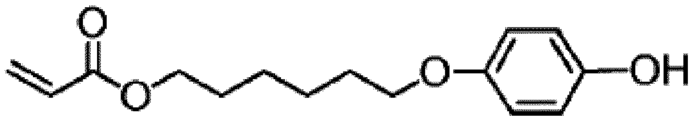

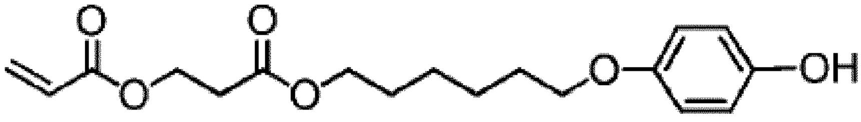

Intermediate mixture A

A three-necked reaction vessel equipped with a thermometer and a condenser having a Dean-Stark tube was charged with 10.00 g of the brown crystals containing the intermediate 9 synthesized in step 9 of Synthesis Example 1, 100 g of toluene, and 0.105 g (0.476 mmol) of 2,6-di-t-butyl-p-cresol in a stream of nitrogen, and the entire contents of the reaction vessel were stirred. The resultant solution was heated to 80°C, 5.14 g (71.3 mmol) of acrylic acid and 0.91 g (9.51 mmol) of methanesulfonic acid were added thereto, and then a dehydration reaction was carried out under reflux conditions (110°C) for 3 hours while removing produced water. Next, the reaction liquid was cooled to 30°C and 500 g of distilled water was added thereto. The entire contents of the reaction vessel were stirred and were then left at rest. The organic layer was separated and 500 g of 5% saline water was added thereto. Liquid separation was performed, and the organic layer was collected and then dried with anhydrous sodium sulfate. Sodium sulfate was subsequently filtered off. Concentrating was performed using a rotary evaporator and then purification was performed by silica gel column chromatography (toluene:ethyl acetate = 8:1 (volume ratio)) to yield 8.2 g of intermediate mixture A in the form of a white solid. The content ratio (molar ratio) of compounds contained in the white solid was determined to be as shown below through analysis of the white solid by high-performance liquid chromatography. This mixture was used in step 2 without purification.

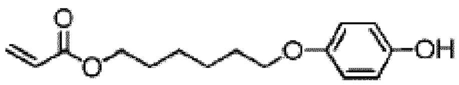

: 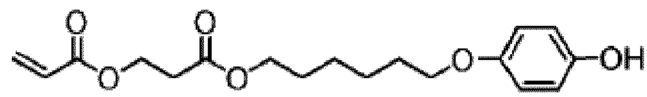 = 9 7 . 5 : 2 . 5

Step 2: Synthesis of intermediate mixture B

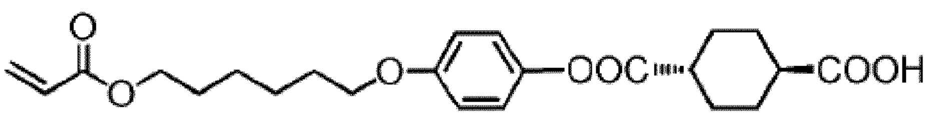

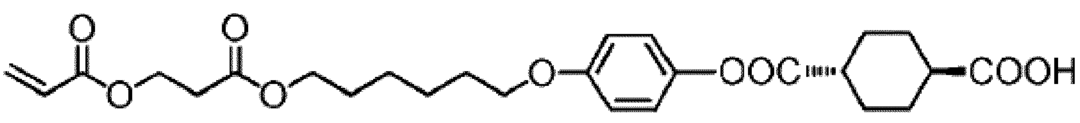

Intermediate mixture B

A three-necked reaction vessel equipped with a thermometer was charged with 10.68 g (62.0 mmol) of trans-1,4-cyclohexanedicarboxylic acid and 90 mL of THF in a stream of nitrogen. In addition, 3.9 g (34.1 mmol) of methanesulfonyl chloride was added into the reaction vessel and the reaction vessel was immersed in a water bath to attain a reaction liquid internal temperature of 20°C. Next, 3.79 g (37.5 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C. Next, 0.38 g (3.12 mmol) of

4-(dimethylamino)pyridine and 8.2 g of the intermediate mixture A synthesized in step 1 were added to the resultant reaction liquid. The reaction vessel was immersed in a water bath once again to attain a reaction liquid internal temperature of 15°C. Next, 3.79 g (37.5 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C. Once the reaction ended, 1,000 mL of distilled water and 100 mL of saturated saline water were added to the reaction liquid, and two extractions were performed with 400 mL of ethyl acetate. The organic layers were collected and were dried with anhydrous sodium sulfate, and then sodium sulfate was filtered off. Solvent was removed from the filtrate by evaporation in a rotary evaporator and then the resultant residue was purified by silica gel column chromatography (THF:toluene = 1:9 (volume ratio)) to yield 7.1 g of intermediate mixture B in the form of a white solid. The content ratio (molar ratio) of compounds contained in the white solid was determined to be as shown below through analysis of the white solid by high-performance liquid chromatography. This mixture was used in step 3 without purification.

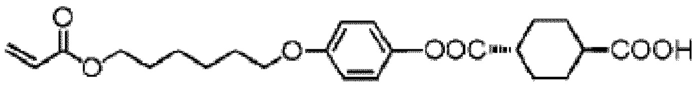

:

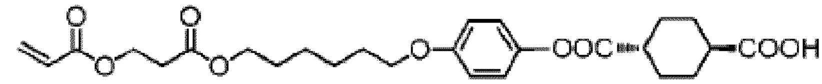

= 98.0 : 2.0

Step 3: Synthesis of mixture 1

A four-necked reaction vessel equipped with a thermometer was charged with 1.0 g (3.21 mmol) of the intermediate 6 synthesized in step 6 of Synthesis Example 1 and 100 mL of chloroform in a stream of nitrogen. Next, 2.96 g of the intermediate mixture B synthesized in step 2 and 39 mg (0.32 mmol) of 4-dimethylaminopyridine were added to the resultant solution. The solution was cooled to 0°C, and then 968 mg (7.67 mmol) of N,N'-diisopropylcarbodiimide was added and was stirred therewith for 1 hour at room temperature. Once the reaction ended, the reaction liquid was filtered using a filter medium precoated with silica gel and was subsequently concentrated under reduced pressure to obtain a residue to which 150 mL of methanol was then added. Precipitated white solid was filtered off and vacuum dried to yield 2.1 g of mixture 1 in the form of a white solid. The content ratio (molar ratio) of compounds contained in the white solid was determined to be as shown below through analysis of the white solid by high-performance liquid chromatography.

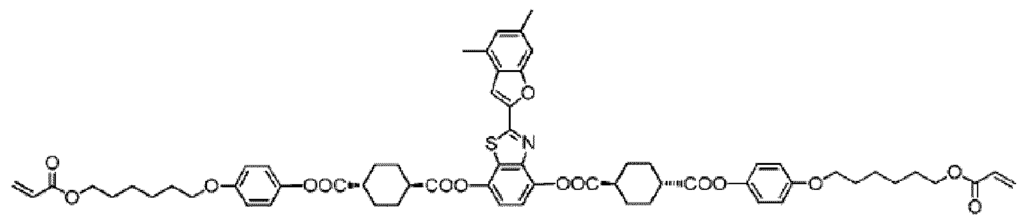

:

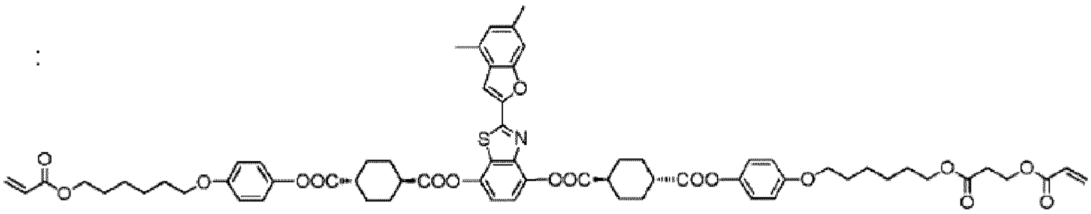

＝９８．７：１．３

(Synthesis Example 8) Synthesis of mixture 2

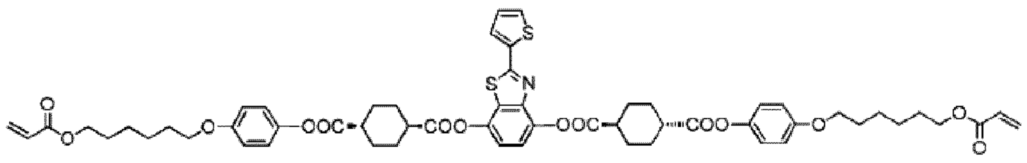

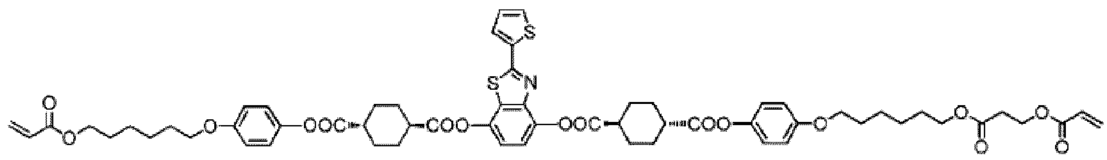

Mixture 2

A four-necked reaction vessel equipped with a thermometer was charged with 1.0 g (4.01 mmol) of the intermediate 15 synthesized in step 4 of Synthesis Example 4 and 100 mL of chloroform in a stream of nitrogen. Next, 3.52 g of the intermediate mixture B synthesized in step 2 of Synthesis Example 7 and 98 mg (0.80 mmol) of 4-dimethylaminopyridine were added to the resultant solution. The solution was cooled to 0°C and then 1.21 g (9.58 mmol) of N,N'-diisopropylcarbodiimide was added and stirred therewith for 1 hour at room temperature. Once the reaction ended, the reaction liquid was filtered using a filter medium precoated with silica gel and was subsequently concentrated under reduced pressure to obtain a residue to which 150 mL of methanol was then added. Precipitated white solid was filtered off and vacuum dried to yield 3.2 g of mixture 2 in the form of a white solid. The content ratio (molar ratio) of compounds contained in the white solid was as shown below.

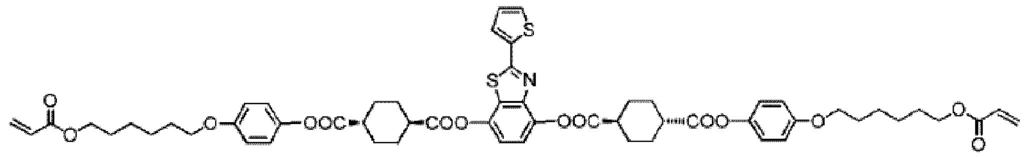

:

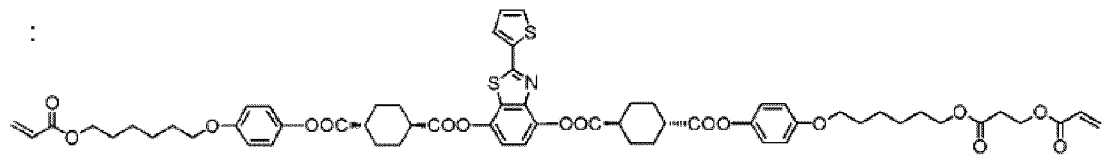

＝９８．６：１．４

(Examples 1 to 17 and Comparative Examples 1 and 2)

Mixtures were prepared by mixing the compounds 1 to 6 and mixtures 1 and 2 obtained in Synthesis Examples 1 to 8, a photoinitiator Irgacure #819 (produced by BASF), a surfactant BYK361N (produced by BYK Additives and Instruments), and cyclopentanone in proportions indicated in Table 1.

Each of the mixtures was heated to 90°C to cause dissolution and was then filtered using a disposable filter having a pore diameter of 0.45 μm while still hot. In this manner, polymerizable liquid crystal compositions 1 to 19 were obtained.

<Evaluation of liquid crystal phase stability>

(i) Formation of liquid crystal layer by polymerizable composition

A #4 wire bar was used to apply each of the polymerizable liquid crystal compositions 1 to 19 onto a transparent glass substrate provided with a polyimide alignment film that had been subjected to rubbing (product name: Alignment Treatment Glass Substrate; produced by E.H.C. Co., Ltd.). The applied film was dried for 1 minute at a temperature indicated below in Table 2 and was then subjected to alignment treatment for 1 minute at a temperature indicated in Table 2 so as to form a liquid crystal layer (thickness: approximately 1.3 μm).

(ii) Formation of optically anisotropic body

The liquid crystal layer prepared in (i) was left for 1 minute or for 15 minutes at a temperature indicated in Table 2. In each case, irradiation with 2000 mJ/$cm^2$ of ultraviolet rays was subsequently performed from the application surface side of the liquid crystal layer at a temperature indicated in Table 2 so as to cause polymerization and thereby obtain an optically anisotropic body attached to a transparent glass substrate.

(iii) Judgment of liquid crystal phase stability

The optically anisotropic body attached to the transparent glass substrate that was obtained in (ii) was positioned as illustrated in FIGS. 1A and 1B, and the surface state thereof was inspected by eye. Note that a state without non-uniformity is a good state. Evaluation was performed by ranking the degree of non-uniformity using five stages with a state in which there is little non-uniformity evaluated as 5 and a state in which non-uniformity arises evaluated as 1. The evaluation results are summarized in Table 2.

Note that PVA polarizing films (produced by Sumitomo Chemical Co., Ltd.) were used as the polarizing films illustrated in FIGS. 1A and 1B. Photographs of a state without non-uniformity (evaluation index: 5) and a state with non-uniformity (evaluation index: 1) are presented in FIGS. 2A and 2B, respectively.

<Measurement of optical properties>

An ellipsometer (model M2000U produced by J. A. Woollam Co., Ltd.) was used to measure retardation between 245.9 nm and 998.4 nm with respect to the prepared optically anisotropic body attached to the transparent glass substrate. Wavelength dispersion was evaluated using α and β values calculated using the measured retardation as shown below.

$$\alpha = (\text{Retardation at 449.9 nm})/(\text{Retardation at 548.5 nm})$$

$$\beta = (\text{Retardation at 650.2 nm})/(\text{Retardation at 548.5 nm})$$

In the case of ideal wavelength dispersion displaying wideband properties (i.e., in a case in which reverse wavelength dispersion is displayed), α is smaller than 1 and β is larger than 1. In the case of flat wavelength dispersion, α and β have roughly the same value. In the case of typical normal dispersion, α is larger than 1 and β is smaller than 1.

In other words, flat wavelength dispersion where α and β are roughly the same value is preferable, and reverse wavelength dispersion where α is smaller than 1 and β is larger than 1 is particularly preferable.

<Measurement of film thickness>

Film thickness was measured by using a needle to form a scratch in the optically anisotropic body of the transparent glass substrate-attached optically anisotropic body, and then measuring a step at the scratch using a surface profiler Dektak 150 (produced by ULVAC, Inc.).

Table 1

| | Polymerizable composition | Polymerizable compound or polymerizable mixture (mass%) | | | | | | | | Polymerization initiator Irgacure #819 (mass%) | Surfactant BYK316N (mass%) | Solvent cyclopentanone (mass%) |
|---|---|---|---|---|---|---|---|---|---|---|---|---|
| | | Compound 1 | Compound 2 | Compound 3 | Compound 4 | Compound 5 | Compound 6 | Mixture 1 | Mixture 2 | | | |
| Example 1 | 1 | 0.1987 | | 19.671 | | | | | | 0.61 | 0.02 | 79.49 |
| Example 2 | 2 | | 0.199 | 19.671 | | | | | | | | |
| Example 3 | 3 | 0.09935 | 0.09935 | 19.671 | | | | | | | | |
| Example 4 | 4 | 1.987 | | 17.883 | | | | | | | | |
| Example 5 | 5 | | 1.987 | 17.883 | | | | | | | | |
| Example 6 | 6 | 0.9935 | 0.9935 | 17.883 | | | | | | | | |
| Example 7 | 7 | 0.1987 | | 14.704 | | | 4.968 | | | | | |
| Example 8 | 8 | | 0.1987 | 14.704 | | | 4.968 | | | | | |
| Example 9 | 9 | 0.09935 | 0.09935 | 14.704 | | | 4.968 | | | | | |
| Example 10 | 10 | 1.987 | | 12.9155 | | | 4.968 | | | | | |
| Example 11 | 11 | | 1.987 | 12.9155 | | | 4.968 | | | | | |
| Example 12 | 12 | 0.9935 | 0.9935 | 12.9155 | | | 4.968 | | | | | |
| Example 13 | 13 | | | 14.903 | 0.9935 | 0.9935 | 2.9805 | | | | | |
| Example 14 | 14 | 19.87 | | | | | | | | | | |
| Example 15 | 15 | | 19.87 | | | | | | | | | |
| Example 16 | 16 | | | | | | | 19.87 | | | | |
| Example 17 | 17 | | | | | | | 14.9025 | 4.9675 | | | |
| Comparative Example 1 | 18 | | | 19.87 | | | | | | | | |
| Comparative Example 2 | 19 | | | 14.9025 | 4.9675 | | | | | | | |

Table 2

| | Polymerizable composition | Drying temperature (°C) | Alignment treatment temperature (°C) | Temperature at which liquid crystal layer is left (°C) | Temperature during photoexposure (°C) | Result of non-uniformity evaluation after being left for 1 minute | Result of non-uniformity evaluation after being left for 15 minutes | Film thickness (μm) | Re (548.5 nm) | α | β |
|---|---|---|---|---|---|---|---|---|---|---|---|
| Example 1 | 1 | 160 | 160 | 150 | 150 | 4 | 4 | 1.348 | 69.61 | 0.796 | 1.054 |
| Example 2 | 2 | 160 | 160 | 150 | 150 | 4 | 3 | 1.351 | 69.76 | 0.797 | 1.056 |
| Example 3 | 3 | 160 | 160 | 150 | 150 | 4 | 4 | 1.350 | 69.71 | 0.797 | 1.055 |
| Example 4 | 4 | 155 | 155 | 145 | 145 | 5 | 5 | 1.353 | 69.87 | 0.799 | 1.058 |
| Example 5 | 5 | 160 | 160 | 150 | 150 | 4 | 4 | 1.349 | 69.66 | 0.796 | 1.055 |
| Example 6 | 6 | 155 | 155 | 145 | 145 | 5 | 5 | 1.355 | 69.97 | 0.800 | 1.059 |
| Example 7 | 7 | 150 | 150 | 140 | 140 | 5 | 4 | 1.358 | 73.96 | 0.846 | 1.034 |
| Example 8 | 8 | 150 | 150 | 140 | 140 | 4 | 4 | 1.354 | 73.74 | 0.843 | 1.031 |
| Example 9 | 9 | 150 | 150 | 140 | 140 | 4 | 4 | 1.354 | 73.74 | 0.843 | 1.031 |
| Example 10 | 10 | 145 | 145 | 135 | 135 | 5 | 5 | 1.356 | 73.85 | 0.845 | 1.033 |
| Example 11 | 11 | 150 | 150 | 140 | 140 | 4 | 4 | 1.355 | 73.79 | 0.844 | 1.032 |
| Example 12 | 12 | 145 | 145 | 135 | 135 | 5 | 5 | 1.358 | 73.96 | 0.846 | 1.034 |
| Example 13 | 13 | 145 | 145 | 135 | 135 | 5 | 4 | 1.359 | 74.01 | 0.846 | 1.035 |
| Example 14 | 14 | 160 | 160 | 150 | 150 | 3 | 3 | 1.344 | 69.40 | 0.793 | 1.051 |
| Example 15 | 15 | 160 | 160 | 150 | 150 | 3 | 3 | 1.349 | 69.66 | 0.796 | 1.055 |
| Example 16 | 16 | 160 | 160 | 150 | 150 | 4 | 4 | 1.346 | 69.51 | 0.794 | 1.052 |
| Example 17 | 17 | 150 | 150 | 140 | 140 | 4 | 4 | 1.345 | 73.25 | 0.838 | 1.024 |
| Comparative Example 1 | 18 | 160 | 160 | 150 | 150 | 3 | 1 | 1.350 | 69.74 | 0.797 | 1.056 |
| Comparative Example 2 | 19 | 150 | 150 | 140 | 140 | 4 | 2 | 1.349 | 73.55 | 0.841 | 1.029 |

INDUSTRIAL APPLICABILITY

The present disclosure provides a polymerizable liquid crystal composition with which an optical film or the like that can maintain a liquid crystal phase over a long period with higher stability, has a practical low melting point, and can perform uniform polarized light conversion over a wide wavelength region can be produced at low cost and with a wide process margin.

Moreover, the present disclosure provides a polymerizable compound and a mixture containing the polymerizable compound that are useful in production of the polymerizable liquid crystal composition.

Furthermore, the present disclosure provides an optical film and an optically anisotropic body that can perform uniform polarized light conversion over a wide wavelength region, and a polarizer, a display, and an antireflection film in which the optical film and the optically anisotropic body are used.

## Claims

1. A polymerizable compound indicated by formula (I), shown below,

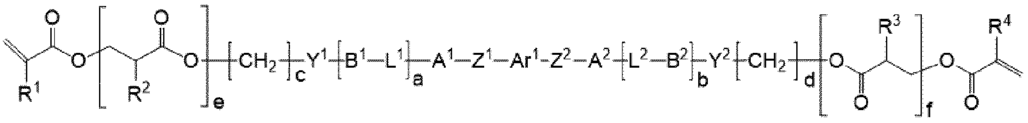

(I)

where, in formula (I), $Ar^1$ is any one of groups represented by formulae (II-1) to (11-4), shown below,

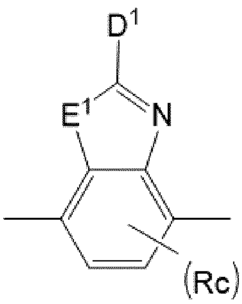

(II-1)

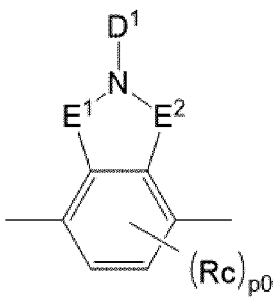

(II-2)

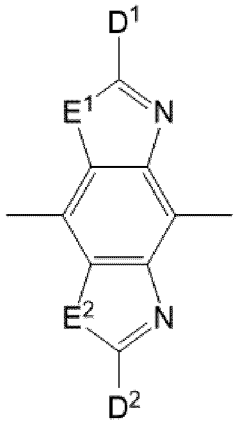

(II-3)

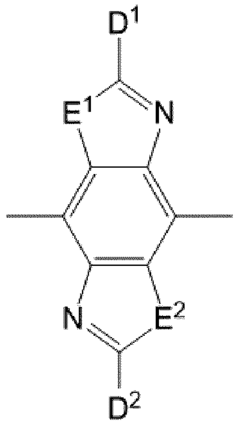

(II-4)

$E^1$ and $E^2$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number of 1 to 4,

Rc represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,

p0 is an integer of 0 to 2,

$D^1$ and $D^2$ each represent, independently of one another, an optionally substituted aromatic hydrocarbon cyclic group or an optionally substituted aromatic heterocyclic group,

$Z^1$ and $Z^2$ each represent, independently of one another, a single bond, $-O-CH_2-$, $-CH_2-O-$, -C(=O)-O-, -O-C(=O)-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C=C-, where

$R^{13}$ represents a hydrogen atom or an alkyl group having a carbon number of 1 to 6,
$A^1$, $A^2$, $B^1$, and $B^2$ each represent, independently of one another, an optionally substituted alicyclic group or an optionally substituted aromatic group,
$Y^1$, $Y^2$, $L^1$, and $L^2$ each represent, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$, where $R^{14}$ and $R^{15}$ each represent, independently of one another, a hydrogen atom or an alkyl group having a carbon number of 1 to 6,
$R^1$ to $R^4$ each represent, independently of one another, a hydrogen atom, a methyl group, or a chlorine atom,
a and b are each, independently of one another, 0 or 1,
one of e and f is an integer of 1 to 3 and the other of e and f is an integer of 0 to 3,
c and d are each, independently of one another, an integer of 1 to 20, and
in a case in which more than one $R^2$, $R^3$, or Rc is present, each $R^2$, $R^3$, or Rc may be the same or different.

**2.** The polymerizable compound according to claim 1, wherein $D^1$ and $D^2$ are each, independently of one another, any one of groups represented by formulae (III-1) to (III-8), shown below,

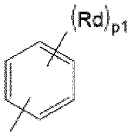
(III-1)
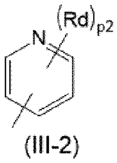
(III-2)
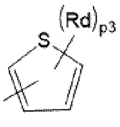
(III-3)
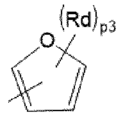
(III-4)
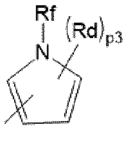
(III-5)
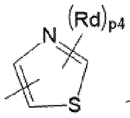
(III-6)
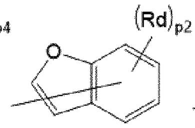
(III-7)
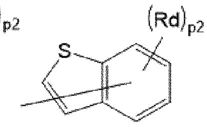
(III-8)

where, in formulae (III-1) to (III-8), Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, p3 represents an integer of 0 to 3, and p4 represents an integer of 0 to 2,
Rf represents a hydrogen atom or a methyl group, and
in a case in which more than one Rd is present, each Rd may be the same or different.

**3.** The polymerizable compound according to claim 1 or 2, wherein $Ar^1$ is any one of groups represented by formulae (IV-1) to (IV-5), shown below,

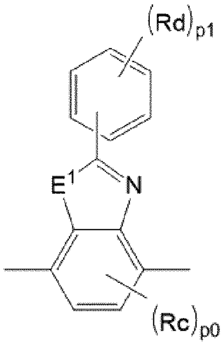
(IV-1)
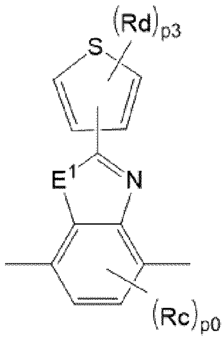
(IV-2)
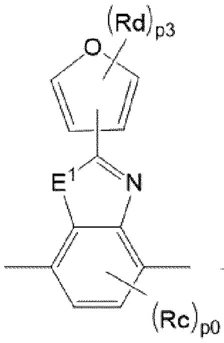
(IV-3)
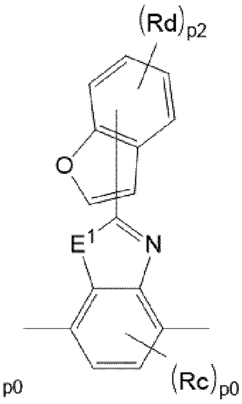
(IV-4)
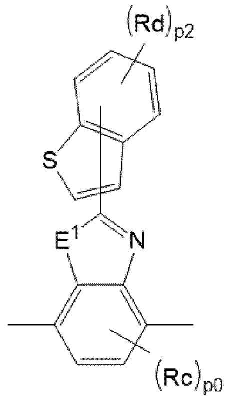
(IV-5)

where, in formulae (IV-1) to (IV-5), $E^1$, Rc, and p0 have the same meaning as previously described,
Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon

number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, and p3 represents an integer of 0 to 3, and in a case in which more than one Rc or Rd is present, each Rc or Rd may be the same or different.

**4.** A mixture comprising:

the polymerizable compound according to any one of claims 1 to 3; and
a polymerizable compound indicated by formula (V), shown below,

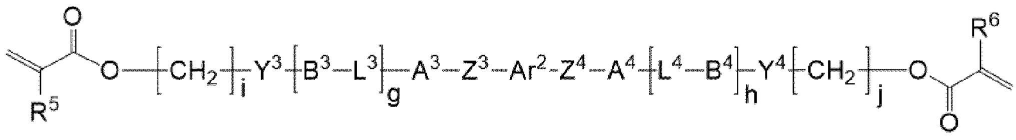

(V)

where, in formula (V), $Ar^2$ is any one of groups represented by formulae (VI-1) to (VI-4), shown below,

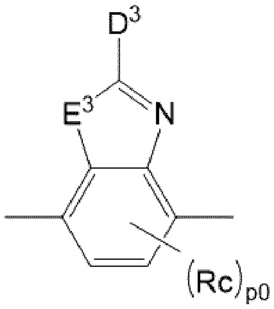

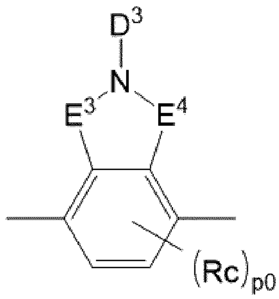

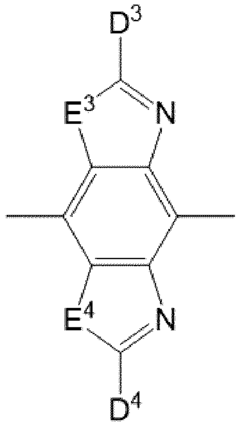

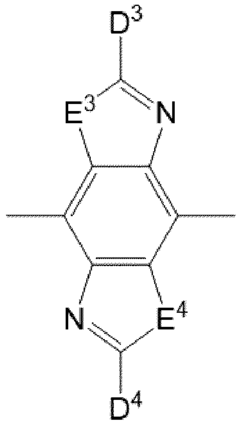

(VI-1) (VI-2) (VI-3) (VI-4)

$E^3$ and $E^4$ each represent, independently of one another, $-CR^{11}R^{12}-$, -S-, $-NR^{11}-$, -CO-, or -O-, where $R^{11}$ and $R^{12}$ each represent, independently of one another, a hydrogen atom or an alkyl group having a carbon number of 1 to 4,
Rc represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p0 is an integer of 0 to 2,
$D^3$ and $D^4$ each represent, independently of one another, an optionally substituted aromatic hydrocarbon cyclic group or an optionally substituted aromatic heterocyclic group,
$Z^3$ and $Z^4$ each represent, independently of one another, a single bond, $-O-CH_2-$, $-CH_2-O-$, -C(=O)-O-, -O-C(=O)-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C=C-, where $R^{13}$ represents a hydrogen atom or an alkyl group having a carbon number of 1 to 6,
$A^3$, $A^4$, $B^3$, and $B^4$ each represent, independently of one another, an optionally substituted alicyclic group or an optionally substituted aromatic group,
$Y^3$, $Y^4$, $L^3$, and $L^4$ each represent, independently of one another, a single bond, -O-, -CO-, -CO-O-, -O-CO-, $-NR^{14}-CO-$, $-CO-NR^{14}-$, -O-CO-O-, $-NR^{14}-CO-O-$, $-O-CO-NR^{14}-$, or $-NR^{14}-CO-NR^{15}-$, where $R^{14}$ and $R^{15}$ each represent, independently of one another, a hydrogen atom or an alkyl group having a carbon number of 1 to 6,
$R^5$ and $R^6$ each represent, independently of one another, a hydrogen atom, a methyl group, or a chlorine atom,

g and h are each, independently of one another, 0 or 1,
i and j are each, independently of one another, an integer of 1 to 20, and
in a case in which more than one Rc is present, each Rc may be the same or different.

**5.** The mixture according to claim 4, wherein $D^3$ and $D^4$ are each, independently of one another, any one of groups represented by formulae (III-1) to (III-8), shown below,

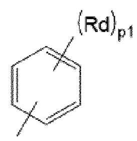
(III-1)
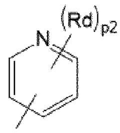
(III-2)
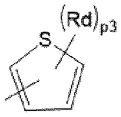
(III-3)
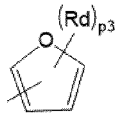
(III-4)
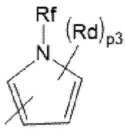
(III-5)
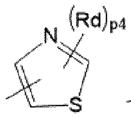
(III-6)
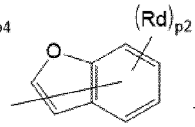
(III-7)
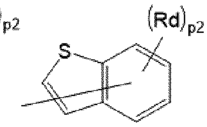
(III-8)

where, in formulae (III-1) to (III-8), Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, p3 represents an integer of 0 to 3, and p4 represents an integer of 0 to 2,
Rf represents a hydrogen atom or a methyl group, and
in a case in which more than one Rd is present, each Rd may be the same or different.

**6.** The mixture according to claim 4 or 5, wherein $Ar^2$ is any one of groups represented by formulae (VII-1) to (VII-5), shown below,

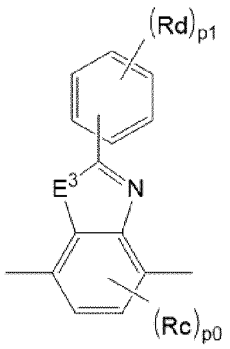
(VII-1)
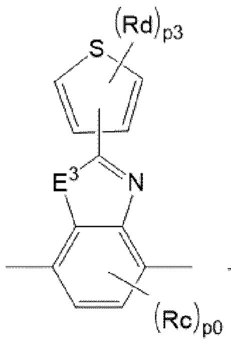
(VII-2)
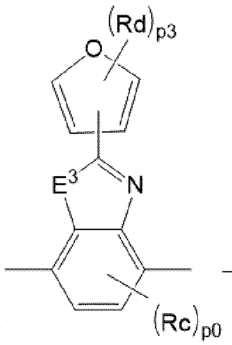
(VII-3)
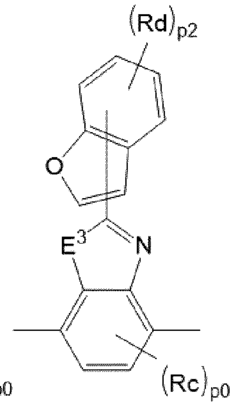
(VII-4)
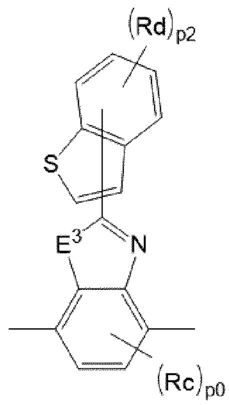
(VII-5)

where, in formulae (VII-1) to (VII-5), $E^3$, Rc, and p0 have the same meaning as previously described,
Rd represents a halogen atom, an alkyl group having a carbon number of 1 to 6, a cyano group, a nitro group, an alkylsulfinyl group having a carbon number of 1 to 6, an alkylsulfonyl group having a carbon number of 1 to 6, a carboxyl group, a fluoroalkyl group having a carbon number of 1 to 6, an alkoxy group having a carbon number of 1 to 6, a thioalkyl group having a carbon number of 1 to 6, an N-alkylamino group having a carbon number of 1 to 6, an N,N-dialkylamino group having a carbon number of 2 to 12, an N-alkylsulfamoyl group having a carbon number of 1 to 6, or an N,N-dialkylsulfamoyl group having a carbon number of 2 to 12,
p1 represents an integer of 0 to 5, p2 represents an integer of 0 to 4, and p3 represents an integer of 0 to 3, and in a case in which more than one Rc or Rd is present, each Rc or Rd may be the same or different.

**7.** The mixture according to any one of claims 4 to 6, wherein a mass ratio of the polymerizable compound indicated by formula (I) relative to the polymerizable compound indicated by formula (V) is 1:1,000 to 20:100.

**8.** A polymerizable liquid crystal composition comprising:

the mixture according to any one of claims 4 to 7; and
a polymerization initiator.

**9.** A polymer obtained by polymerizing the mixture according to any one of claims 4 to 7 or the polymerizable liquid crystal composition according to claim 8.

**10.** An optical film comprising the polymer according to claim 9 as a constituent material.

**11.** An optically anisotropic body comprising a layer having the polymer according to claim 9 as a constituent material.

**12.** A polarizer comprising:

the optically anisotropic body according to claim 11; and
a polarizing film.

**13.** A display comprising the polarizer according to claim 12.

**14.** An antireflection film comprising the polarizer according to claim 12.

FIG. 1A

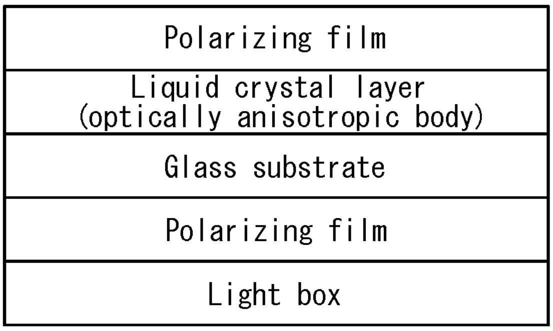
Polarizing film
Liquid crystal layer
(optically anisotropic body)
Glass substrate
Polarizing film
Light box

FIG. 1B

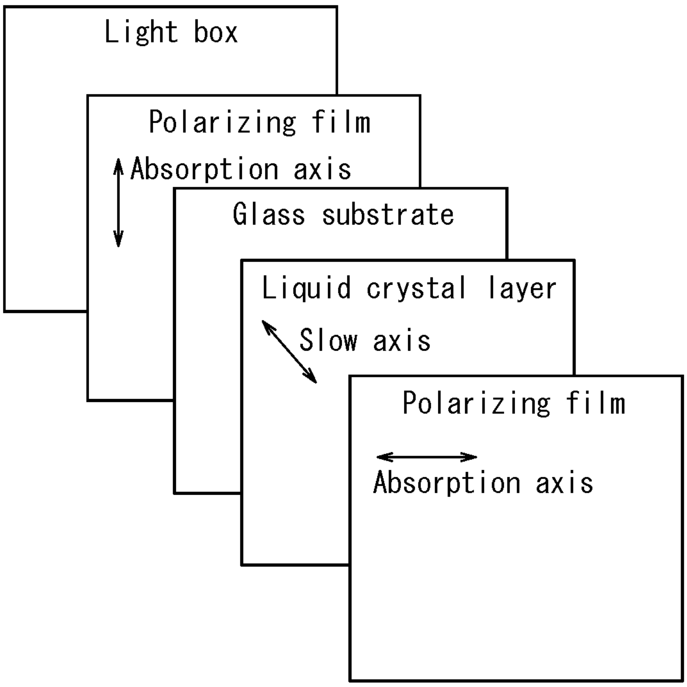
Light box
Polarizing film
Absorption axis
Glass substrate
Liquid crystal layer
Slow axis
Polarizing film
Absorption axis

FIG. 2A

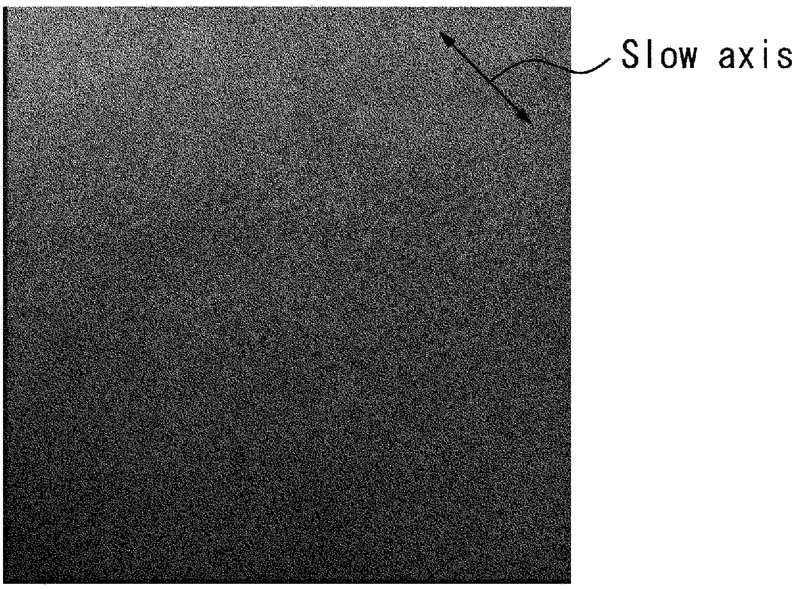


FIG. 2B

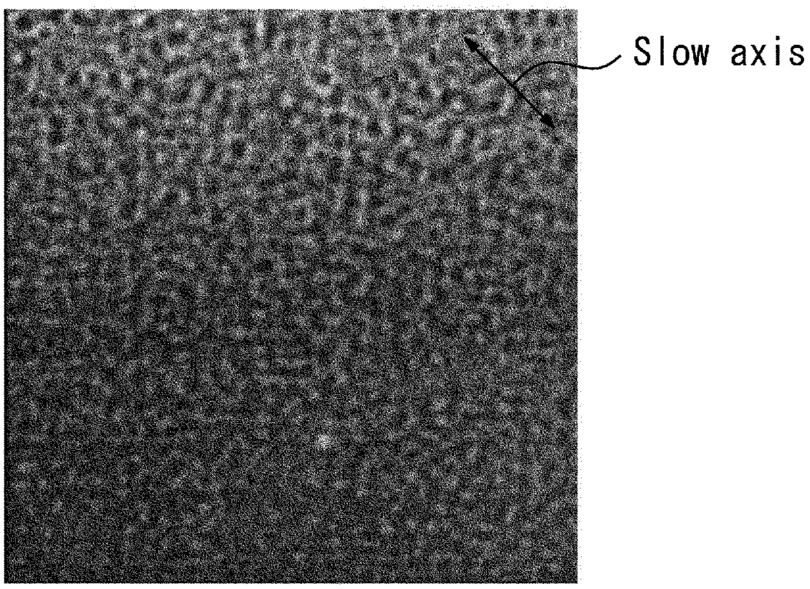

**INTERNATIONAL SEARCH REPORT**

International application No.
PCT/JP2017/040656

A. CLASSIFICATION OF SUBJECT MATTER
Int.Cl. C08F20/34(2006.01)i, C08F2/48(2006.01)i, C08F20/38(2006.01)i, C08F220/34(2006.01)i, C08F220/38(2006.01)i, G02B1/11(2015.01)i, G02B5/30(2006.01)i

According to International Patent Classification (IPC) or to both national classification and IPC

B. FIELDS SEARCHED

Minimum documentation searched (classification system followed by classification symbols)
Int.Cl. C08C19/00-19/44, C08F6/00-246/00, C08F301/00, C08F2/48, G02B1/11, G02B5/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2015/080219 A1 (DIC CORPORATION) 04 June 2015, entire text & US 2017/0002123 A1, entire text | 1-14 |
| A | JP 2009-173893 A (SUMITOMO CHEMICAL CO., LTD.) 06 August 2009, entire text (Family: none) | 1-14 |
| A | JP 2011-006361 A (SUMITOMO CHEMICAL CO., LTD.) 13 January 2011, entire text (Family: none) | 1-14 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 February 2018 (05.02.2018) | 13 February 2018 (13.02.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/040656

C (Continuation). DOCUMENTS CONSIDERED TO BE RELEVANT

| Category* | Citation of document, with indication, where appropriate, of the relevant passages | Relevant to claim No. |
|---|---|---|
| A | JP 2011-042606 A (SUMITOMO CHEMICAL CO., LTD.) 03 March 2011, entire text (Family: none) | 1-14 |
| A | JP 2011-162678 A (SUMITOMO CHEMICAL CO., LTD.) 25 August 2011, entire text (Family: none) | 1-14 |
| P, A | JP 6055569 B1 (NIPPON ZEON CO., LTD.) 09 December 2016, entire text (Family: none) | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A1
- JP 2015200877 A
- JP 2010031223 A
- JP 2007002208 A
- JP 2009173893 A
- JP 2009274984 A
- JP 2010030979 A
- JP 2011006360 A
- JP 2010024438 A
- JP H05310845 A
- US 5179171 A
- JP H0597978 A
- US 5202388 A
- JP H11124429 A
- WO 9920676 A1


### Non-patent literature cited in the description


- March's Advanced Organic Chemistry. Wiley
- **SANDLER ; KARO.** Syntheses of Organic Compounds Classified by Functional Group. Naoki INAMOTO